# EUROPEAN PATENT APPLICATION

(11) **EP 0 897 755 A2**
(43) Date of publication of application: **24.02.1999**
(21) Application number: 98306421.3
(22) Date of filing: 12.08.1998
(51) Int. Cl.: B05B 17/06

(54) **Piezoelectric chemical-liquid atomizer apparatus and method for repelling or eliminating harmful organism**

(30) Priority: 20.08.1997 JP 224117/97; 04.12.1997 JP 334400/97; 09.03.1998 JP 55636/98
(71) Applicant: FUMAKILLA LIMITED, Tokyo (JP)
(72) Inventor: Abe, Toshio, Hatsukaichi-shi, Hiroshima-ken (JP); Yamazaki, Satoshi, Hatsukaichi-shi, Hiroshima-ken (JP); Sugiura, Masaaki, Saika-gun, Hiroshima-ken (JP); Orita, Kunitaka, Saika-gun, Hiroshima-ken (JP)
(74) Representative: Marchant, James Ian

(57) **Abstract**

The piezoelectric chemical-liquid atomiser apparatus (11) including a chemical liquid vessel (12) detachably housed in the atomiser apparatus (11), and a wick for supplying a chemical liquid therethrough to a piezoelectric atomising head arranged in the atomiser apparatus (11), characterized in that the wick is divided into a first chemical-liquid passage portion and a second chemical-liquid passage portion (4), wherein (A) the first chemical-liquid passage portion is arranged within the chemical liquid vessel (12), one end thereof contacting the chemical liquid and the other end thereof being abutted against one end of the second chemical-liquid passage portion (3), and (B) the second chemical-liquid passage portion (3) is arranged at a position where the other end thereof slightly touches the piezoelectric atomising head or a position where the other end thereof contacts the piezoelectric atomising head, thereby allowing the chemical liquid to be supplied up to the piezoelectric atomising head through the first chemical-liquid passage portion (4) and the second chemical-liquid passage portion (3). The above piezoelectric chemical-liquid atomiser apparatus is highly useful for a method for repelling and eliminating a harmful organism.

## Description

### BACKGROUND OF THE INVENTION

### Field of the Invention

The present invention relates generally to a piezoelectric chemical-liquid atomiser apparatus for spraying chemical liquids for eliminating insects, eliminating mites, retarding insects' growth, repelling insects, perfuming, deodorizing, disinfecting, and the like. The present invention further relates to a method for repelling or eliminating harmful organisms by efficiently and economically emitting in the air an effective ingredient having functions of eliminating insects, eliminating mites, retarding insects' growth and repelling insects.

### Discussion of the Related Art

There have been utilized methods using mosquito repellent incense or mosquito-repellent mats and liquid heating transpiration apparatuses as conventional methods for emitting chemical liquids in the air for the purpose of eliminating insects, and the like. These methods have merits and demerits, respectively and therefore, a suitable method is selected therefrom that meets a utilizable situation or a utilizable period. More recently, the method of utilizing the liquid heating transpiration apparatus has been used widely because of its merit that the transpiration apparatus does not require replenishment of chemical liquid for a long period of time.

Any of these methods, however, are adapted for transpiration the effective ingredient with heating. For instance, the temperature of a heating portion may rise as high as 100°C or more, which risks danger. In some cases, chemical liquids contain flammable kerosine as a solvent. Further, these methods of transpiration of the chemical liquids with heating have a detrimental tendency that the transpiration amount of the chemical liquid decreases with passage of time. Particularly in a transpiration apparatus arranged such that a wick draws up a chemical liquid, and a part of the wick is heated, the heated portion of the wick tends to be clogged by such causation as deterioration of the effective ingredient with heating. In this connection, there has been an urgent need for development of a technique for stably emitting the chemical liquid in the air without using a heating means.

As a liquid atomising method without utilizing heat, there has been disclosed a liquid atomising technique using a piezoelectric actuator (see, for example, Japanese Unexamined Patent Publication No. Hei 7-501481). In the method using a so-called piezoelectric atomiser apparatus as mentioned above, spray particles are generated by feeding a liquid to be sprayed to a vibrating portion vibrated at a high frequency. Methods for feeding the liquid to the vibrating portion include, for instance, a method of contacting or slightly touching a liquid-retaining member (a wick) impregnated with the liquid with a vibrating portion (see, for example, Japanese Patent Laid-Open Nos. Hei 5-329411 and Hei 6-320083).

In the apparatus in which the liquid is fed to the vibrating portion by means of the wick as described above, a gap between the vibrating portion and the wick or a state of contact therebetween is extremely important.

For instance, Japanese Unexamined Patent Publication No. Hei 7-501481 discloses an apparatus for producing fine liquid droplets in which a liquid is directly fed to a film used as a diaphragm by means of a capillary liquid feeder or the like. In addition, Japanese Patent Laid-Open No. Hei 5-329411 discloses a liquid feeder for use in an ultrasonic atomiser apparatus having a structure that porous diaphragm is securely fixed to a piezoelectric vibrator. Further, Japanese Patent Laid-Open No. Hei 6-320083 discloses an ultrasonic atomiser apparatus having a structure that an atomising end of a liquid retaining member is brought into contact with a diaphragm by a loading means.

Particularly in the piezoelectric atomisation techniques disclosed in the above-mentioned three patent publications, in a case where a gap is provided with a distance wide enough not to cause slight touch between the diaphragm and the wick, the chemical liquid cannot be smoothly fed to the diaphragm and hence, the spray particles are not formed. On the other hand, in a case where the contact between the diaphragm and the wick is too strong, the wick may interfere with desired vibration of the diaphragm or reduce the service life of the diaphragm. Therefore, in the piezoelectric atomiser apparatus, it is desirable that a constant gap or an unchanged state of contact between the piezoelectric atomising head (e.g., diaphragm) and the wick is always maintained regardless of the serviceable conditions.

In the piezoelectric atomiser apparatus utilizing the wick for feeding a liquid to a piezoelectric atomising head (e.g., diaphragm), there has been known that a vessel (chemical liquid vessel) for storing the liquid in a body of the apparatus is incorporated or detachably housed in the body of the apparatus. In the case where the chemical liquid vessel is incorporated in the body of the apparatus, an operation for replenishing the chemical liquid is very cumbersome, and there is a danger that an operator's hand may be exposed to a spilled chemical liquid during the replenishing operation. In the case where the chemical liquid vessel is detachably housed in the apparatus body, there have been proposed the following two embodiments: One in which only the chemical liquid vessels are exchanged while the wick remains housed in the apparatus body; and the other in which the wick is exchanged at the same time of exchange of the liquid vessel. In the case of one embodiment, since the chemical liquid vessel is open at a portion for housing the wick, there is the same danger with the aforesaid instance that the operator's hand may be exposed to a spilled chemical liquid. In the case of the other embodiment, there is a great concern about unwanted variations in the gap or the state of contact between the diaphragm and the wick, which the unwanted variation may result from dimensional variations of a member constituting the liquid vessel and human factors such as a manner to house the liquid vessel to the apparatus.

On the other hand, as to the method for repelling or eliminating harmful organisms by emitting an effective ingredient in the air, there have conventionally been known a method using a liquid heating transpiration apparatus; a method utilizing a so-called aerosol method comprising atomising a chemical liquid by discharging a high-pressure gas together with the chemical liquid; and a method utilizing a so-called piezoelectric method comprising atomising a chemical liquid through ultrasonic waves requiring high output, and the like.

Among these methods, recently, the method using a liquid heating transpiration apparatus, in particular, has been well used because of its convenience in not requiring replenishment of liquid for a long period of time. In this method, since the chemical liquid containing the effective ingredient is discharged by continuously heating the liquid, once the heating temperature is stabilized, the chemical liquid particles can be continuously fed in the air. The size of the chemical liquid particles discharged from the heating transpiration apparatus described above are extremely small, and the particles relatively quickly become floating and diffusing in the heat-ascending air flow. Particularly, a tightly closed room allows the chemical liquid particles to stay floating for a longer period of time. Together with the aforesaid continuous feeding of the chemical liquid particles, this method provides an advantage of a long-lasting effect for repelling or eliminating the harmful organisms.

On the other hand, there are the following disadvantages. Since the chemical liquid is discharged through indirect continuous heating thereof, a great amount of time must be taken between the start of use and the exhibition of the effects of the chemical liquid. Furthermore, since the method involves continuous heat generation, requiring high energy, it is practically impossible to use a battery or the like to drive the transpiration apparatus for a long period of time. Further, since the heated portion of the wick tends to be clogged by such causation as deterioration of the effective ingredient with heating, the transpiration amount of the chemical liquid decreases in the latter half of the service period. In view of the above, in order to achieve power saving, for instance, if the transpiration apparatus is intermittently driven, a long time is required until the effect of the chemical liquid is exhibited through a transpiration portion heated to a predetermined temperature, and hence, a desired effect or a desired power saving cannot be accomplished.

By contrast, the method for discharging the chemical liquid by the aerosol method does not require electrical energy for atomisation energy of the chemical liquid, and allows instantaneous discharge of the chemical liquid in large amounts. Unfortunately, however, the presently commercially available aerosol products require the manual push-button operation, and besides, a given amount of chemical liquid cannot be discharged without the use of a special constant flow valve. Furthermore, many of the aerosol products are designed for principally treating the spraying particles directly upon target pests. In consideration of transcutaneous penetration of the effective ingredient into insect bodies, the aerosol products generally has a large particle size. Therefore, there are the following disadvantages when the particle size is large: 1) Slow diffusion rate in the air; 2) fast falling velocities of the particles; and 3) polluting the region where the chemical liquid is applied.

One object of the present invention is to provide a piezoelectric chemical-liquid atomiser apparatus with easy chemical liquid exchange operation, having substantially no liquid leakage when exchanging the chemical liquid, and reducing the variation in the gap or state of contact between the piezoelectric atomising head comprising a diaphragm, a piezoelectric actuator, and the like, and the wick, thereby improving the atomising stability.

One object of the present invention is to provide a method for repelling or eliminating a harmful organism capable of reducing energy required for releasing the chemical liquid for repelling or eliminating a harmful organism, having a high safety, and being stable for a long period of time.

These and other objects of the present invention will be apparent from the following description.

### SUMMARY OF THE INVENTION

In one aspect, the present invention pertains to a piezoelectric chemical-liquid atomiser apparatus comprising:
a chemical liquid vessel detachably housed in the atomiser apparatus, and
a wick for supplying a chemical liquid therethrough to a piezoelectric atomising head arranged in the atomiser apparatus,
characterized in that:
the wick is divided into a first chemical-liquid passage portion and a second chemical-liquid passage portion, wherein
(A) the first chemical-liquid passage portion is arranged within the chemical liquid vessel, one end thereof contacting the chemical liquid and the other end thereof being abutted against one end of the second chemical-liquid passage portion; and
(B) the second chemical-liquid passage portion is arranged at a position where the other end thereof slightly touches the piezoelectric atomising head or a position where the other end thereof contacts the piezoelectric atomising head, thereby allowing the chemical liquid to be supplied up to the piezoelectric atomising head through the first chemical-liquid passage portion and the second chemical-liquid passage portion.

In another aspect, the present invention pertains to a method for repelling and eliminating a harmful organism comprising emitting in the air a chemical liquid containing an effective ingredient as atomised chemical liquid fine particles, characterized in that the atomised chemical liquid is sprayed intermittently, and to have a particle size distribution of the resulting atomized chemical liquid fine particles in which 90% by cumulative volume of the chemical liquid fine particles based on volume cumulative distribution has a particle size of 20 µm or less.

### BRIEF DESCRIPTION OF THE DRAWINGS

The present invention will become more fully understood from the detailed description given hereinbelow and the accompanying drawings which are given by way of illustration only, and thus, are not limitative of the present invention, and wherein:
Figure 1 is a schematic view illustrating an exemplary construction of a diaphragm, a piezoelectric actuator, a second chemical-liquid passage portion, and a first chemical-liquid passage portion;
Figure 2 is a schematic view illustrating a construction of a piezoelectric chemical-liquid atomiser apparatus in one embodiment of the present invention, which is a cross-sectional view of an atomiser apparatus;
Figure 3 is a schematic view illustrating a construction of a piezoelectric chemical-liquid atomiser apparatus in another embodiment of the present invention, which is a cross-sectional view of an atomiser apparatus;
Figure 4 is a schematic view illustrating a chemical-liquid atomiser apparatus for the aerosol method including a control mechanism of open-close of the atomising head, and a control mechanism of spraying time interval and amount of sprayed liquid; and
Figure 5 is a schematic view illustrating a piezoelectric chemical-liquid atomiser apparatus.

In the figures, 1 denotes a diaphragm, 2 a piezoelectric actuator, 3 a second chemical-liquid passage portion, 4 a first chemical-liquid passage portion, 5 a fixing piece, 6 a fixing member, 7 a metal mesh, 11 an atomiser apparatus, 12 a chemical liquid vessel, 13 a rotatable cover, 14 a chemical liquid, 15 a piezoelectric actuator, 16 a battery, 17 a packing member, 18 a vent, 19 a spray opening, 20 a slidable switch, 21 a battery cover, 32 a propellant, 33 a dip tube, 34 a pressure vessel, 35 an atomising head, 36 a button, 37 an electromagnetic valve, 38 an electromagnetic-valve control circuit, 39 a timer circuit, 41 an emitting port, 55 a wick, and 56 an oscillation control circuit.

### DETAILED DESCRIPTION OF THE INVENTION

A piezoelectric chemical-liquid atomiser apparatus of the present invention comprises:
a chemical liquid vessel detachably housed in the atomiser apparatus, and
a wick for supplying a chemical liquid therethrough to a piezoelectric atomising head arranged in the atomiser apparatus,
characterized in that:
the wick is divided into a first chemical-liquid passage portion and a second chemical-liquid passage portion, wherein
(A) the first chemical-liquid passage portion is arranged within the chemical liquid vessel, one end thereof contacting the chemical liquid and the other end thereof being abutted against one end of the second chemical-liquid passage portion; and
(B) the second chemical-liquid passage portion is arranged at a position where the other end thereof slightly touches the piezoelectric atomising head or a position where the other end thereof contacts the piezoelectric atomising head, thereby allowing the chemical liquid to be supplied up to the piezoelectric atomising head through the first chemical-liquid passage portion and the second chemical-liquid passage portion.

In the piezoelectric chemical-liquid atomiser apparatus of the present invention, the wick is divided into the first chemical-liquid passage portion and the second chemical-liquid passage portion. The chemical liquid vessel is independent from and detachably housed in the apparatus body, wherein the first chemical-liquid passage portion is arranged within the chemical liquid vessel, and the second chemical-liquid passage portion is arranged in the apparatus body. Since the first chemical-liquid passage portion is arranged within the chemical liquid vessel, the position of the second chemical-liquid passage portion is not changed when mounting or detaching the chemical liquid vessel. Therefore, a given state of contact between the other end of the second chemical-liquid passage portion and the piezoelectric atomising head is maintained without affecting the mounting or detaching the chemical liquid vessel.

The chemical liquid vessel in the present invention is so constructed as to be detachably housed in the atomiser apparatus and includes the first chemical-liquid passage portion therein. The first chemical-liquid passage portion serves as a medium for absorbing the chemical liquid in the chemical liquid vessel and conveying the absorbed chemical liquid to the second chemical-liquid passage portion, and also as a simple plug for preventing the chemical liquid from being spilled even when the chemical liquid vessel turns over on its side. Hence, the first chemical-liquid passage portion is arranged within the chemical liquid vessel, one end thereof contacting the chemical liquid in the vessel, and the other end thereof being abutted against one end of the second chemical-liquid passage portion.

The chemical liquid vessel of the chemical-liquid atomiser apparatus according to the present invention may have a vent (aperture) having a hole area of 1 mm² or less at a position higher than a liquid level of the chemical liquid. The vent serves to maintain a pressure within the chemical liquid vessel at the same level as the external pressure, and functions as a member for providing stable supply of the chemical liquid to the second chemical-liquid passage portion and consequently to the piezoelectric atomising head and functions as a member for preventing liquid leakage from the vessel during storage. Hence, it is preferred that the chemical-liquid atomiser apparatus includes the chemical liquid vessel having such a vent. Generally, in the chemical liquid vessel without a vent, a rise in the air temperature, an approach of a low atmospheric pressure or the like may sometimes cause the pressure in the vessels to rise relatively to the external pressure, resulting in causing phenomena such as an extreme increase in the supply amount of the chemical liquid from a first chemical-liquid passage portion and a large amount of chemical liquid overflow. Hence, the aforesaid vent having an aperture of 1 mm² or less is useful for the prevention of these phenomena. A preferable aperture area of the vent is 1 mm² or less, from the viewpoint of preventing the liquid leakage from the vent when the vessel is turned over.

Further, it is preferred that the chemical-liquid atomiser apparatus according to the present invention may further comprise a packing member in the periphery of the abutment portion of the first chemical-liquid passage portion against the second chemical-liquid passage portion. The packing member may be attached to a side of the liquid vessel or to a side of the apparatus body. A manner to attach the packing member is not particularly limited. By including such a packing member at the abutment portion, it is preferable that the periphery of contact points between the chemical-liquid passage portions is kept air tight, thereby preventing runs or drips of the liquid.

The atomiser apparatus in the present invention has the second chemical-liquid passage portion arranged at a position where the other end thereof slightly touches the piezoelectric atomising head in the atomiser apparatus or a position where the other end thereof contacts the piezoelectric atomising head. The atomiser apparatus body further includes a piezoelectric actuator, an oscillation circuit and the like which are generally included in the known piezoelectric atomiser apparatuses. The shape of the piezoelectric atomising head is not particularly limited, and it may take any one of the typical ones known in the art. For example, there can be preferably used a piezoelectric atomising head having a construction such that a porous diaphragm is secured to a piezoelectric actuator in a direct or indirect manner. The direction in which the piezoelectric atomising head is mounted is not limited to a horizontal position relative to the floor (i.e. to spray in an upward direction), and it may be positioned in an arbitrary angle. Incidentally, the diaphragms and piezoelectric actuators usable in the present invention include any one of those generally known in the art.

Specific examples of a member which slightly touches the other end of the second chemical-liquid passage portion or contacts the other end of the second chemical-liquid passage portion include a diaphragm, a thin sheet, a piezoelectric actuator, and the like. The diaphragm and thin sheet may, for example, be porous, mesh-like, or the like.

Particularly in the chemical-liquid atomiser apparatus comprising the piezoelectric atomising head including a diaphragm, the state of contact between the piezoelectric atomising head and the other end of the second chemical-liquid passage portion is preferably to a degree that the other end of the second chemical-liquid passage portion slightly touches the piezoelectric atomising head. The term "slightly touches" used herein means a state in which the other end of the second chemical-liquid passage portion so softly touches the diaphragm or piezoelectric actuator as not to interfere with the vibration thereof; or it means a state in which such a fine gap is formed between the other end of the second chemical-liquid passage portion and the diaphragm or piezoelectric actuator that a film of the chemical liquid formed on the top surface of the other end of the second chemical-liquid passage portion barely contacts the piezoelectric atomising head, such as the diaphragm. By the slight touch between the piezoelectric atomising head and the other end of the second chemical-liquid passage portion, the chemical liquid from the second chemical-liquid passage portion to the piezoelectric atomising head can be stably supplied without the interference with the vibration of the diaphragm or the piezoelectric actuator for atomising the chemical liquid.

The size of the "fine gap" for allowing the aforesaid slight touch depends upon the shape of the piezoelectric atomising head or the thickness of the chemical liquid film formed on the top surface of the second chemical-liquid passage portion. Since the film thickness, in particular, is affected by a surface tension of the chemical liquid and a surface energy of the second chemical-liquid passage portion, it is impossible to make a sweeping statement about this gap. However, for instance, the minimum gap is preferably the minimum vibrational amplitude of a porous diaphragm, and the maximum gap is preferably 0.5 mm or less, more preferably 0.3 mm or less, and particularly preferably 0.1 mm or less.

In the slight touch, where the piezoelectric atomising head and the second chemical-liquid passage portion are in the "state of softly touching," the second chemical-liquid passage portion is more preferably made of a relatively flexible material.

In the chemical-liquid atomiser apparatus in an embodiment where the piezoelectric atomising head includes a porous or mesh-like thin sheet, the state of contact between the piezoelectric atomising head and the other end of the second chemical-liquid passage portion is to a degree that the other end of the passage portion contacts the piezoelectric atomising head. A concrete example of the piezoelectric atomising head in this embodiment includes a porous or mesh-like thin sheet of a circular shape being arranged at a side where the chemical liquid is discharged (e.g., upper surface) of a disk-like piezoelectric actuator, and circumferential portions of these are integrally retained by a flexible annular fixing member while the other end of the second chemical-liquid passage portion contacts a part of the circumferential portions thus retained. In this case, the side where the chemical liquid is discharged of the piezoelectric actuator and the lower surface of the thin sheet form a fine gap therebetween, and the chemical liquid spreads onto the fine gap supplied from the second chemical-liquid passage portion. Thus, the stable supply of the chemical liquid can be provided by the ensured contact between the other end of the second chemical-liquid passage portion and the piezoelectric atomising head.

As described in the foregoing, the gap or the state of contact between the piezoelectric atomising head and the second chemical-liquid passage portion is extremely important. Accordingly, it is desirable that the second chemical-liquid passage portion is stably fixed in the atomiser apparatus body at all times in order to ensure that this gap or state of contact may not be changed even when a chemical liquid vessel is housed in the atomiser apparatus thereby to bring the first chemical-liquid passage portion into abutment against the second chemical-liquid passage portion.

In the piezoelectric chemical-liquid atomiser apparatus according to the present invention, the first chemical-liquid passage portion is arranged within the chemical liquid vessel, one end thereof contacting the chemical liquid and the other end thereof abutted against the one end of the second chemical-liquid passage portion, while the second chemical-liquid passage portion is arranged where the other end thereof slightly touches the piezoelectric atomising head or where the other end thereof contacts the piezoelectric atomising head. Therefore, the chemical liquid is supplied from the chemical liquid vessel through the first chemical-liquid passage portion and the second chemical-liquid passage portion up to the piezoelectric atomising head.

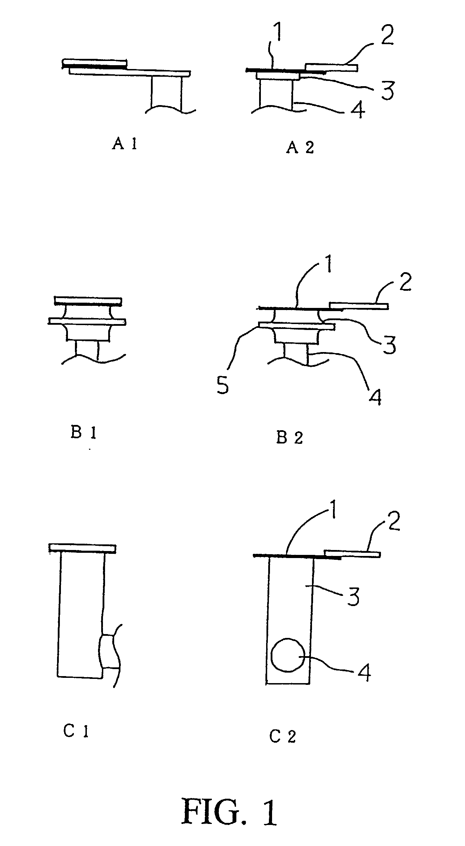

Figure 1 illustrates an exemplary construction of the present invention comprising a diaphragm 1, a piezoelectric actuator 2, a second chemical-liquid passage portion 3 and a first chemical-liquid passage portion 4. In Figure 1, the illustration of the parts other than the diaphragm 1, the piezoelectric actuator 2, the second chemical-liquid passage portion 3, the first chemical-liquid passage portion 4 and a fixing piece 5 are omitted for the sake of simplicity.

In Figure 1, A1 and A2 show an embodiment where a position in which a first chemical-liquid passage portion 4 is abutted against a second chemical-liquid passage portion 3 is shifted in a horizontal direction from the position where the second chemical-liquid passage portion 3 slightly touches a diaphragm 1. This embodiment has an advantage that even when the first chemical-liquid passage portion 4 is abutted against the second chemical-liquid passage portion 3 from below, a stress generated owing to the abutment is not directly applied to the diaphragm 1, so that the gap or state of contact between the second chemical-liquid passage portion 3 and the diaphragm 1 is not affected (or changed). Incidentally, in Figure 1, A1 is a front view, and A2 is a side view.

In Figure 1, B1 and B2 show an embodiment where a second chemical-liquid passage portion 3 is made of a relatively hard, porous material, and a fixing piece 5 is securely fixed to the vessel body. This embodiment has an advantage that the second chemical-liquid passage portion 3 is made of a relatively hard material, so that the position of the second chemical-liquid passage portion 3 is not changed in the atomiser apparatus, even when applied with a load owing to the abutment from the first chemical-liquid passage portion 4. Incidentally, in Figure 1, B1 is a front view, and B2 is a side view.

In Figure 1, C1 and C2 show an embodiment where the abutment of a first chemical-liquid passage portion 4 against a second chemical-liquid passage portion 3 is in a different direction from that in which the second chemical-liquid passage portion 3 slightly touches a diaphragm 1. This embodiment has an advantage that stress generated owing to the abutment from the first chemical-liquid passage portion 4 does not directly affect the direction in which the second chemical-liquid passage portion 3 slightly touches a diaphragm 1, so that a degree of slight touch therebetween is maintained in an unchanged state. Incidentally, in Figure 1, C1 is a front view, and C2 is a side view.

In the present specification, it is preferred that the abutment pressure of the first chemical-liquid passage portion to the second chemical-liquid passage portion is 200 g/cm² or less. In order to stably feed the chemical liquid from the first chemical-liquid passage portion to the second chemical-liquid passage portion, it is particularly preferred that the abutment pressure is suitably set in the range from 0.1 to 100 g/cm², depending upon the abutment direction or construction of the chemical-liquid passage portions.

In the present invention, an embodiment where at least one of the first chemical-liquid passage portion and the second chemical-liquid passage portions is compressed by the abutment between these chemical-liquid passage portion is preferable. By the compression, there is expected an effect that the state of contact between these first and second chemical-liquid passage portions is made stable, and pores are locally formed, so that the wicking speed of the chemical liquid is increased. Furthermore, such pores are expected to serve as a filter for preventing foreign substances in the chemical liquid from being migrated, even if such foreign substances were contained therein.

The filtering effect is particularly useful in a case where, for example, the diaphragm of the piezoelectric atomising head is formed with a large number of pores having diameters of 10 µm or less for spraying fine chemical liquid particles. The pores formed in the chemical-liquid passage portions may have diameters ranging from about 0.5 to 30 µm and are not necessarily required to be smaller than the size of the pores formed in the diaphragm. The diameters of such pores formed in the chemical-liquid passage portions may be determined by appropriately compressing a member employed for the chemical-liquid passage portion, and taking measurement on an enlarged cut surface of the compressed member through a microscope or the like.

As described in the foregoing, since the wick is divided into the first chemical-liquid passage portion and the second chemical-liquid passage portion, the replenishment of the chemical liquid or exchange of the chemical liquid vessels can be facilitated, and a gap or state of contact between the piezoelectric atomising head and the wick (the second chemical-liquid passage portion) can be made at a constant level. More specifically, there can be levelled variations, which may change the gap or state of contact between the piezoelectric atomising head and the wick, in the size of components constituting the chemical liquid vessel and in the mounting position of the housed chemical liquid vessels. Thus, more easy production control of the chemical liquid vessels can be achieved.

Materials for the first chemical-liquid passage portion and the second chemical-liquid passage portion usable in the present invention are preferably exemplified by porous materials having continuously permeable pores, resinous materials having continuous foams, or aggregates of resinous fibers. Concrete examples thereof include resinous materials having continuous foams, such as polyurethanes, polyethylenes, polyethylene terephthalates, polyvinyl formals, and polystyrenes; porous materials prepared by tableting with sintering resinous fine particles of, for instance, polyethylene, polypropylene, nylon, or the like as a main component; porous materials made of poly(ethylene fluorides); felt materials, such as polyesters, polypropylene, nylon, acrylic, rayon, and wool; aggregates of resinous fibers such as nonwoven fabrics made of such fibers as polyolefin fibers, polyester fibers, nylon fibers, rayon fibers, acrylic fibers, vinylon fibers, polyfural fibers, and aramid fibers; porous, powder inorganic sintered body prepared by tableting with sintering inorganic powders of, for instance, ceramics as a main component, without being limited thereto. In addition, these materials for the first and second chemical-liquid passage portions may be treated with a surfactant. Further, the materials for the first chemical-liquid passage portion may be the same as or different from those for the second chemical-liquid passage portion.

Since each of the first chemical-liquid passage portion and the second chemical-liquid passage portion serves as a medium for supplying a chemical liquid to the piezoelectric atomising head, the materials used therefor having high liquid permeability are preferable. Concretely, the materials used for the first chemical-liquid passage portion having a wicking speed of the chemical liquid of 10 minutes or less are preferable, more preferably 5 minutes or less. It is still more preferred that in addition to satisfy the above wicking speed, those having an ability of wicking the chemical liquid to a height of 40 mm or higher of the chemical-liquid passage portion are satisfied, particularly preferably 50 mm or higher. In the present invention, the term "wicking speed of the chemical liquid" means a time for a chemical liquid to reach a height of 30 mm above the liquid level after immersing a chemical-liquid passage portion having dimensions of 5 mm in width, 5 mm in thickness, and 60 mm in length to a position 10 mm from the bottom portion at room temperature 25°C. In addition, the term "ability of wicking the chemical liquid" means a height of the chemical liquid reached after 60 minutes from initiation of immersion, as measured by the same method as the wicking speed of the chemical liquid described above.

Here, in a case of using such materials as nonwoven fabrics as the chemical-liquid passage portion, where the thickness needed for measurement cannot be obtained, the thickness is not particularly defined as long as the materials have 5 mm in width and 70 mm in length. The wicking speed of the chemical liquid and the ability of wicking the chemical liquid are measured by using the chemical liquid to be atomised.

Next, the member used for the second chemical-liquid passage portion preferably satisfies the same conditions as the first chemical-liquid passage portion. However, in practical terms, the member for the second chemical-liquid passage portion only need to supply the chemical liquid from the first chemical-liquid passage portion to the piezoelectric atomising head in a stable manner, and the wicking speed of the chemical liquid and the ability of wicking the chemical liquid are not particularly specified.

It is preferred that the piezoelectric atomising head and the second chemical-liquid passage always maintain a constant gap or state of contact therebetween, regardless of conditions of use and the like. Therefore, the second chemical-liquid passage portion preferably exhibits a smaller degree of swell when the second chemical-liquid passage portion is impregnated with the chemical liquid.

In the present invention, the first chemical-liquid passage portion and the second chemical-liquid passage portion serving as the wick preferably have higher feeding speeds for the chemical liquid, from the viewpoint of efficiently generating sprayed particles. It is common practice in the art to set the feeding speed for the chemical liquid of the wick to a level equal to or faster than a spraying speed for the chemical liquid of the piezoelectric atomising head. However, as a technique for increasing accuracy of the amount of sprayed chemical liquid, the feeding speed for the chemical liquid of the first chemical-liquid passage portion and/or the feeding speed of the second chemical-liquid passage portion may be set to a lower level than the spraying speed for the chemical liquid, thereby to control the amount of sprayed chemical liquid. This technique is effective for an atomising apparatus which repetitively atomise on a relatively short time basis. The technique controls the supply amount of the chemical liquid through the chemical-liquid passage portions as well as mechanical or electric control of the amount of sprayed liquid, so that it is desirable for improving the accuracy of a spraying amount per one wisp of spray, or a spraying amount of the chemical liquid per unit time period.

In the present invention, a method for housing the chemical liquid vessel in the atomiser apparatus is not particularly limited, as long as the method allows detachable housing of the chemical liquid vessel in the atomiser apparatus. In a case where a chemical liquid vessel is housed in an atomiser apparatus, the method allows that one end of the first chemical-liquid passage portion is brought into abutment against one end of the second chemical-liquid passage portion. Examples of a useful method for housing include a method comprising horizontally shifting a chemical liquid vessel into the apparatus body from a lateral side of the chemical liquid vessel and fitting the chemical liquid vessel in an atomiser apparatus body (lateral slide-fit method); a method for fitting a chemical liquid vessel in an apparatus body from a lateral side thereof with turning the vessel at a slight angle (lateral snap-fit method); a method for fitting a chemical liquid vessel in an atomiser apparatus body (from top side) in a substantially vertical direction (top-down housing method); a method for fitting a chemical liquid vessel in the atomiser apparatus body (from bottom side) in a substantially vertical direction (bottom-top housing method) and the like. Among these housing methods, the lateral slide-fit method, the lateral snap-fit method and the top-down housing method are more preferred, since it is made unnecessary to carry out such procedures as lifting up the apparatus body for housing or detaching the chemical liquid vessel, these methods provide a simple and easy vessel changing procedure.

Next, the apparatus of the present invention will be described by referring to the drawings.

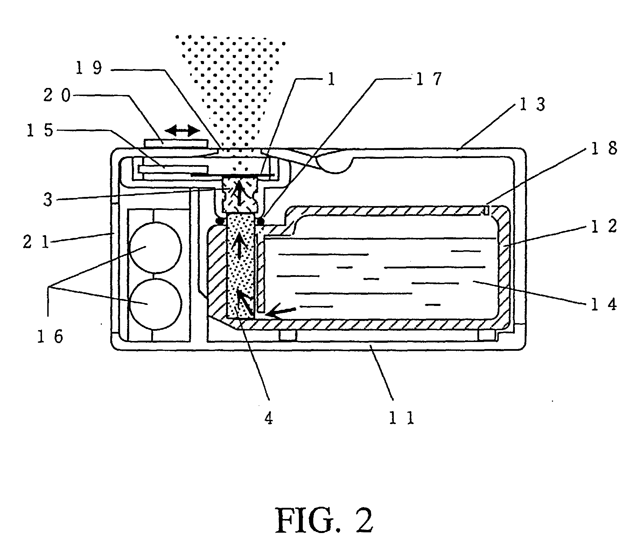

Figure 2 is a schematic view illustrating a construction of a piezoelectric chemical-liquid atomiser apparatus in one embodiment of the present invention, which is a cross-sectional view of an atomiser apparatus. In an atomiser apparatus 11, there is provided a chemical liquid vessel 12. The chemical liquid vessel 12 is detachably housed in the atomiser apparatus 11, and the chemical liquid 12 is taken in and out the atomiser apparatus 11 by opening a rotatable cover 13 attached to the atomiser apparatus 11. In addition, the member for a wick is divided into a first chemical-liquid passage portion 4 and a second chemical-liquid passage portion 3. When the chemical liquid vessel 12 is housed at a given position in the atomiser apparatus 11, the other end of the first chemical-liquid passage portion 4 is brought in abutment against one end of the second chemical-liquid passage portion 3, thereby exhibiting its function as a wick. Here, the chemical liquid vessel 12 is housed by a lateral snap-fit method.

The chemical liquid vessel 12 is provided with the first chemical-liquid passage portion 4, one end thereof contacting a chemical liquid 14. Further, the other end of the first chemical-liquid passage portion 4 is brought into abutment against one end of the second chemical-liquid passage portion 3 housed in the atomiser apparatus 11.

The atomiser apparatus 11 comprises a piezoelectric actuator 15 and a diaphragm 1 attached to the piezoelectric actuator 15, wherein the diaphragm comprises a large number of pores in a regular arrangement. Here, the other end of the second chemical-liquid passage portion 3 slightly touches the diaphragm 1.

In the apparatus of Figure 2, a battery 16 is used as a power source. The battery 16 is taken in and out of the atomiser apparatus 11 by opening a battery cover 21. In addition, although not illustrated in the figure, the atomiser apparatus further comprises an oscillation control circuit connected to the piezoelectric actuator 15, the oscillation control circuit having a function of piezoelectric actuation control and a function of a timer control. In the periphery of the portion where the first chemical-liquid passage portion 4 is brought into abutment against the second chemical-liquid passage portion 3, there is provided a packing member 17. In the chemical liquid vessel 12, a vent 18 is arranged at a position higher than the liquid level of the chemical liquid 14.

By sliding a slidable switch 20 to a position shown in the figure, a spray opening 19 is opened, and the spraying operation is initiated.

The chemical liquid supplied from the chemical liquid vessel 12 passes through the first chemical-liquid passage portion 4 and the second chemical-liquid passage portion 3, and supplied to the diaphragm 1 slightly touching the other end of the second chemical-liquid passage portion 3 as shown by arrows in the figure. By the vibration of the diaphragm 1, the chemical liquid is sprayed through an atomising head 19.

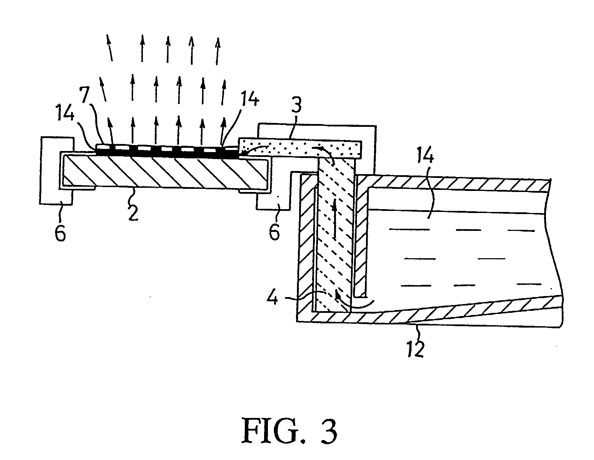

Figure 3 is a cross-sectional view for schematically illustrating a construction of an atomiser apparatus in another embodiment of the present invention.

In this embodiment, a piezoelectric actuator 2 and a metal mesh 7 as a thin sheet arranged thereabove form a fine gap, and a chemical liquid is supplied from a second chemical-liquid passage portion 3 to the fine gap.

As shown by arrows in the figure, the chemical liquid from a chemical liquid vessel 12 penetrates through a first chemical-liquid passage portion 4 and the second chemical-liquid passage portion 3 to be delivered from the other end of the second chemical-liquid passage portion 3 onto a top surface of the piezoelectric actuator 2, and the chemical liquid is introduced into the aforesaid fine gap to spread thereacross. The chemical liquid enters individual pores provided on the metal mesh so as to form fine liquid columns which are atomised and sprayed by the vibration of the piezoelectric actuator in a direction of the thickness of the actuator.

The method for repelling and eliminating a harmful organism of the present invention comprising emitting in the air a chemical liquid containing an effective ingredient as atomised chemical liquid fine particles, is characterized in that the atomised chemical liquid is sprayed intermittently, and to have a particle size distribution of the resulting atomized chemical liquid fine particles in which 90% by cumulative volume of the chemical liquid fine particles based on volume cumulative distribution has a particle size of 20 µm or less. In the present invention, the chemical liquid is emitted in the air as fine liquid droplets (fine chemical liquid particles) by any one of various atomising means, so that the harmful organism may be repelled or eliminated by exhibition of an effect of effective ingredients in the chemical liquid.

As to the diameter of the fine chemical liquid particles, 90% by cumulative volume or more of the chemical liquid fine particles have a diameter of 20 µm or less, more preferably 90% by cumulative volume or more of the chemical liquid fine particles have a diameter of 15 µm or less, still more preferably 90% by cumulative volume or more of the chemical liquid fine particles have a diameter of 10 µm or less.

In the present specification, the diameter of the fine chemical liquid particles herein is a value determined by a particle size distribution analyser by means of a laser beam scattering at 25°C.

In this case, a suitable position for spraying the chemical liquid is set for measuring the diameter of the fine chemical liquid particles by taking into account a spraying output and the like such that the particle size distribution analyser may be allowed to provide favorable measurements. A suitable spraying position is spaced 10 to 50 cm away from the laser beam. It is suitable to measure the particle sizes immediately after the spraying, i.e. within three seconds from the spraying of the chemical liquid.

However, in a case where the use of the particle size distribution analyser cannot provide favorable measurements, such measurement values may be replaced by those given by a classifying device utilizing an Andersen sampler or the like.

In order to intermittently spray fine chemical liquid particles of diameters within a given range, for instance, the following spraying method may be employed.

For instance, a spraying method which utilizes a chemical-liquid atomiser apparatus using the aerosol method, the apparatus comprising a pressure vessel including an openable spray opening, and a chemical liquid sealed in the pressure vessel together with a propellant can be employed. Preferable method of spraying the chemical liquid in the air is a method which utilizes a chemical-liquid atomiser apparatus further including a power source, an open/close control mechanism for the spray opening and a control mechanism of spraying time interval and amount of sprayed liquid, including an electromagnetic valve, a constant rate valve, and the like. In this case, the aforesaid pressure vessel seals in the chemical liquid together with the propellant, the chemical liquid consisting essentially of the effective ingredients or comprising a mixture of effective ingredients and a solvent. The size of the fine chemical liquid particles can be adjusted by mainly adjusting a ratio of an initial volume of the chemical liquid to the overall volume of the pressure vessel.

Alternatively, the piezoelectric spraying method includes a method for spraying the chemical liquid in the air using a piezoelectric atomiser apparatus, including a piezoelectric actuator, a porous diaphragm attached to the piezoelectric actuator, a chemical-liquid feeding means for supplying the chemical liquid to the diaphragm, a piezoelectric oscillation circuit connected to the piezoelectric actuator, a control circuit capable of controlling vibration of the diaphragm for intermittently atomising the chemical liquid, a power source, and a chemical liquid. In this case, the size of the fine chemical liquid particles can mainly be adjusted by adjusting the size of pores provided on the diaphragm. Incidentally, the piezoelectric chemical-liquid atomiser apparatus of the present invention may favorably be used for the spraying method in the present invention.

The chemical liquid usable in the present invention is not particularly limited as long as the chemical liquid contains an effective ingredient exhibiting such effects as to kill insects or mites, retard insects' growth, repel insects, and the like. Alternatively, the effective ingredient itself may be used as the chemical liquid. Examples of effective ingredient usable herein include pyrethroid insecticides, pyrethroid-like insecticides, organic phosphorus insecticides, carbamate insecticides, chloronicotine insecticides, insect growth retardants, and microbicides. In the present invention, these effective ingredients may be used alone or in admixture thereof. In many of these effective ingredients, there are present geometric isomers ascribed to the carboxylic acid component and optical isomers ascribed to asymmetric carbon atom in the carboxylic acid component or the alcohol component. In the present invention, the chemical liquid can contain any of these isomers and an admixture thereof. Concrete examples of the effective ingredient are listed as below.

Examples of the pyrethroid insecticides include allethrin, d1•d-T80-allethrin, d1•d-T-allethrin, d•d-T-allethrin, d•d-T80-prallethrin, phthalthrin, d-T80-phthalthrin, resmethrin, d-T80-resmethrin, d-T80-furamethrin, permethrin, phenothrin, fenvalerate, cypermethrin, d-T80-cyphenothrin, empenthrin, terallethrin, imiprothrin and the like.

Examples of the pyrethroid-like insecticides include Etofenprox and the like.

Examples of the organic phosphorus insecticides include Diazinon, fenitrothion, pyridafenthion, malathion, Dipterex, chlorpyrifos, fenthion, dichlorvos, propetanphos, Abate, prothiophos, phoxim and the like.

Examples of the carbamate insecticides include propoxur and the like.

Examples of the chloronicotine insecticides include imidacloprid, acetamiprorid and the like.

Examples of the insect growth retardants (IGR) include pyriproxyfen, cyromazine and the like.

Examples of the microbicides include sulfur (S), Daconil, MBC, Topsin-M, Sumilex and the like.

Miticides, repellents and other insectifuges may be employed depending upon its purposes.

Examples of the miticide include kelthane and the like.

Examples of the repellent include
N,N-diethyl-m-toluamide, dimethyl phthalate, dibutyl phthalate, 2-ethyl-1,3-hexanediol, p-dichlorobenzene, and the like.

These effective ingredients may be used alone or in admixtures of two or more kinds.

The chemical liquid can be prepared by dissolving or mixing the above effective ingredient in a solvent.

Examples of the solvent usable in the present invention include aliphatic hydrocarbons, alicyclic hydrocarbons, aromatic hydrocarbons, halogenated hydrocarbons, alcohols, esters, ethers, and ketones, with a particular preference given to aliphatic saturated hydrocarbons having 5 to 18 carbon atoms. Incidentally, although the aliphatic unsaturated hydrocarbons are not desirable as a main component of the solvent owing to its unpleasant odor, there is no problem in adding the aliphatic unsaturated hydrocarbons to the aliphatic saturated hydrocarbon in an amount so as not to cause an unpleasant odor.

In addition, in the aliphatic saturated hydrocarbons, there is a tendency that the larger the number of carbon atoms, the more viscous the hydrocarbons, and in some cases, having a gel-like state or solid state at an ambient temperature. In such cases, since mal-effects are likely to be obtained when supplied or sprayed as a chemical liquid, the aliphatic saturated hydrocarbon used as a solvent preferably has 18 or less carbon atoms. From the viewpoint of flash point at an ambient temperature, the aliphatic saturated hydrocarbon used as a solvent preferably has 5 or more carbon atoms. In addition, as to the aliphatic unsaturated hydrocarbons, those having 10 to 14 carbon atoms are more preferable, and particularly those having 11 to 13 carbon atoms are more preferable.

Examples of commercially available solvent comprising these aliphatic saturated hydrocarbons as a main component include "No. O Solvent M" (manufactured by NIPPON OIL CO., LTD.), "No. 0 Solvent L" (manufactured by NIPPON OIL CO., LTD.), "No. 0 Solvent H" (manufactured by NIPPON OIL CO., LTD.), "NORMAL PARAFFIN YH-NP" (manufactured by NIKKO PETROCHEMICALS CO., LTD.), "NORMAL PARAFFIN SH-NP" (manufactured by NIKKO PETROCHEMICALS CO., LTD.), "DEOTOMIZOL A-1" (manufactured by Yoshitomi Pharmaceutical Industries, Ltd.), "IP SOLVENT 2028" (manufactured by IDEMITSU PETROCHEMICAL CO., LTD.), "IP SOLVENT 1620" (manufactured by IDEMITSU PETROCHEMICAL CO., LTD.), "IP SOLVENT 1016" (manufactured by IDEMITSU PETROCHEMICAL CO., LTD.), and "NEO-CHIOZOL" (manufactured by Sanko Kagaku Kogyo K.K.).

Moreover, it is extremely effective to use fats and oil produced mainly extracted from plants and animals and utilize as food additives and starting cosmetic materials from the viewpoint of toxicity. Particularly, those oils which are less likely to be solidified by oxidation, including semi-dry oils such as sesame oil, rapeseed oil, and soybean oil; and those oils which are not solidified including non-dry oils such as camellia oil and olive oil are preferable.

In addition, a composition prepared by mixing one or more compounds selected from the group consisting of alcohols, aldehydes, ketone, and carboxylic acids as a solvent with water together with an effective ingredient may be used as a chemical liquid, or alternatively, a composition prepared by emulsifying and suspending in water the effective ingredient together with a surfactant may be used a chemical liquid. Since the above composition contains water, there is less danger against fire.

Incidentally, there may be other components besides the effective ingredients and solvents included in the chemical liquid. For example, in addition to the above effective ingredients, other components include stabilizing agents such as BHT and 2,2'-methylenebis(4-ethyl-6-t-butylphenol); synergists such as 6-propylpiperonyl ether, octachlorodipropyl ether, isobornyl thiocyan acetate, N-octylbicycloheptene carboxiimide, and N-(2-ethylhexyl)-1-isopropyl-4-methylenebicyclo(2,2,2)oct-5-en-2,3-carboxiimide; animal-derived or plant-derived natural perfumes; artificial perfumes made of hydrocarbons, alcohols, phenols, aldehydes, ketones, lactones, oxides, esters, and like; bactericidalantifungal agents such as O-phenylphenol, isopropylmethylphenol, 2-chloro-4-phenylphenol, and thymol.

The amount of sprayed effective ingredient as contained in the chemical liquid is not particularly limited the present invention. The amount of effective ingredient is suitably specified depending upon a target harmful organism, a type of effective ingredient used and an expected effect. The target harmful organisms include flies, mosquitoes, cockroaches, fleas, mites, moth flies, centipedes, ants, aphids, spider mites, and the like. The target harmful organisms include fungi such as *Erysiphales, Melanconiaceae, Sclerotiniaceae, Moniliaceae, Tuberculariaceae* and the like.

The preferred amount of sprayed effective ingredient in the chemical liquid ranges from 0.01 to 20 mg/h per 30 m³. The amount of sprayed effective ingredient more preferably ranges from 0.05 to 20 mg/h per 30 m³, and particularly preferably ranges from 0.1 to 15 mg/h per 30 m³.

By way of an example, where d•d-T80-prallethrin is used as an effective ingredient for preventing mosquitoes from biting or eliminating them, the effective ingredient is preferably used in amounts in a range from 0.04 to 3.0 mg/h per 30 m³, and more preferably from 0.1 to 1.0 mg/h per 30 m³. Where another effective ingredient is used for the same purpose, the amount thereof is suitably selected from a range from 0.01 to 20 mg/h per 30 m³ depending upon a degree of activity of the effective ingredient used.

In order to set a desired amount of the effective ingredient to be sprayed, a spraying time, a spraying time interval, a size of fine chemical liquid particles and an amount of the chemical liquid emitted by one spray may be adjusted.

In the present invention, the chemical liquid is intermittently sprayed in the air and a stable effect to repel or eliminate harmful organisms is ensured over an extended period of time.

As described in the foregoing, in the present invention, by setting the particle size such that those having a particle size of 20 µm or less, based on volume cumulative distribution (volume cumulative percent), account for 90% or more of the entire particles, the falling velocity of the fine particles and the amount of fallen particles are decreased, thereby allowing the particles to be diffused in a wider area and also ensuring that the effective ingredient stays while floating in the air for a longer time period.

Additionally, the sprayed fine chemical liquid particles exhibit a behavior to gradually decrease in size because a main solvent evaporates from individual particles floating in the air. This leads to an extended floating time of the particles, i.e. an extended duration of effective period and an increased diffusibility of the particles in the air. The tendency of the particles decreasing in size varies depending upon the types of solvent and propellant used and therefore, a suitable solvent and propellant can be selected as desired.

If a group of particles greater than 20 µm in size is present in a volume cumulative percent of more than 10%, an amount of particles quick to fall increases and besides, such particles fall before exhibiting the aforesaid behavior to decrease in size while staying afloat in the air. This results in a lowered concentration of the effective ingredient in air (concentration in air) as well as in a decreased floatability or diffusibility of thereof and hence, a desired effect cannot be attained. In addition, there is increased pollution of a region where the chemical liquid is applied.

In a space where the harmful organism in the present invention is repelled or eliminated, the space including a house (residence), bungalow, tent, shop, greenhouse, vinyl house, warehouse and the like, there is much air flow so that a favorable diffusibility of the effective ingredient is ensured by producing chemical liquid particles of a size specified by the present invention. Furthermore, by the aforesaid effect to extend the floating time of the particles, the continuous atomisation of the chemical liquid is not necessitated. Thus, energy consumption is reduced through intermittent atomisation of the chemical liquid, and moreover, safety is enhanced. The method for repelling or eliminating harmful organism of the present invention is quite advantageous in that an effect as indicated by the effective ingredient is safely and efficiently exhibited and maintained over an extended period of time in a power-saving manner. Therefore, the atomiser apparatus used in the method of the present invention can operate on a battery, such as dry batteries, so that the portability thereof is remarkably facilitated. As a result, the freedom of application of the present invention is dramatically increased.

The method of the present invention adapted for intermittent atomisation gives improvements to the problems of the conventional methods: the aerosol method suffering substantial chemical liquid loss due to greater particle sizes of the sprayed liquid obtained by the method; the liquid-heating transpiration method requiring high energy for continuous transpiration of the liquid even though the resultant particles are small in size; and the ultrasonic atomisation method requiring high energy for instantaneously atomising a large amount of liquid even though the resultant particles are small in size.

In the case of the intermittent atomisation, a time interval between a spray and the next spray (spraying interval) is not particularly limited. The time interval, for example, preferably ranges from 3 seconds to 60 minutes, more preferably from 10 seconds to 30 minutes, and particularly preferably from 20 seconds to 15 minutes. From the viewpoint of energy saving, the time interval of more than 3 seconds is preferred. From the viewpoints of safety and sustainability of the floating chemical liquid particles, the time interval of less than 60 minutes is preferred.

A spraying time for each spray is not particularly limited. However, in consideration of power saving when the battery or the like is used as a power source, the spraying time, for instance, preferably ranges from 0.05 to 300 seconds, more preferably from 0.1 to 180 seconds, and particularly preferably from 0.2 to 60 seconds.

A chemical-liquid atomiser apparatus usable for the method of the present invention is preferably capable of instantaneously emitting a predetermined amount of the chemical liquid. Examples of a preferred atomiser apparatus include the aerosol atomiser apparatus and the piezoelectric atomiser apparatus as mentioned above. The piezoelectric atomiser apparatus of the present invention described above is included in the examples of the preferred piezoelectric atomiser apparatus.

A concrete description will hereinbelow be made on the atomising methods.

### Aerosol Method

In this method, the size of fine chemical liquid particles can be designed to have a predetermined range mainly by adjusting the volume ratio of a chemical liquid to the overall volume of a pressure vessel, the chemical liquid consisting essentially of the effective ingredient, the chemical liquid comprising the effective ingredient and a solvent. More specifically, an initial volume of the chemical liquid preferably accounts for 15% or less of the overall volume of the pressure vessel, and more preferably 10% or less, and particularly preferably 5% or less. Limiting the initial volume ratio of the chemical liquid to 15% or less leaves an initial volume ratio of 85% or more for a propellant occupying the pressure vessel. This provides the fine chemical liquid particles further reduced in size. The propellant may be composed of a gas phase and a liquid phase or of a gas phase alone. In a case where the propellant is composed of the gas phase and liquid phase, it is preferred that a single homogeneous liquid phase is formed by uniformly dissolving or dispersing the chemical liquid in the liquid phase of the propellant. In a case where the propellant is composed of the gas phase alone, it is preferred that the effective ingredient is uniformly dissolved or dispersed in the chemical liquid.

In a case where the chemical liquid is atomised by this method, the chemical liquid may consist essentially of the effective ingredient, or it may comprise a mixture of the effective ingredient and a solvent. In a case where the chemical liquid comprises the above mixture, a concentration of the chemical liquid may optionally be adjusted so that the initial volume ratio of the chemical liquid to the volume of the pressure vessel is within a predetermined range.

A preferred propellant sealed within the pressure vessel include, for example, at least one selected from the group consisting of liquefied petroleum gas (LPG), dimethyl ether (DME) and halogenated hydrocarbons. However, the propellant is not particularly limited to the above and compressed carbon dioxide gas, compressed nitrogen gas, compressed air or the like may be used as long as the desired fine chemical liquid particles can be formed.

The pressure vessel with the openable atomising head usable in the present invention is not particularly limited. Similar vessels to those used for the conventional aerosol products can be used.

As a control mechanism of open-close of the atomising head and the control mechanism of spraying interval and amount of the sprayed liquid, there may be included, for example, a method for electrically controlling both the amount of sprayed liquid and the spraying interval by using an electromagnetic valve adapted to open the atomising head for a given time period, and a method for electrically controlling only the spraying interval, while controlling the amount of sprayed liquid by utilizing a constant rate valve.

By using such a chemical-liquid atomiser apparatus for the aerosol method, the chemical liquid can be automatically and intermittently sprayed.

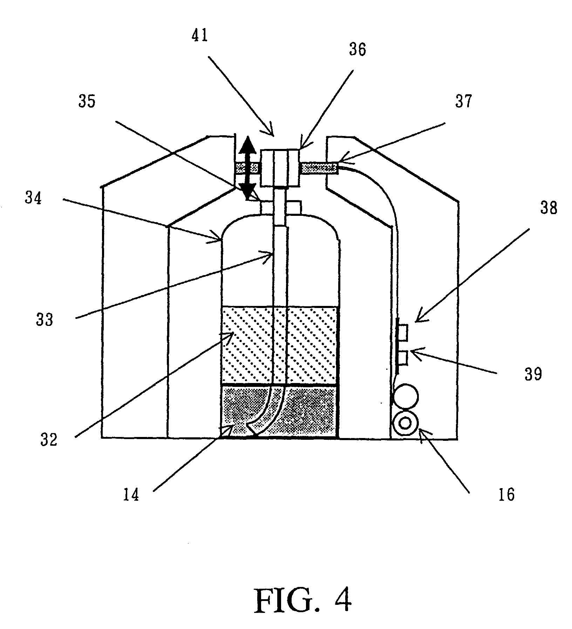

Figure 4 is a schematic view illustrating a chemical-liquid atomiser apparatus for the aerosol method including a power source, a control mechanism of open-close of the atomising head, and a control mechanism of spraying time interval and amount of sprayed liquid. In Figure 4, the apparatus comprises a chemical liquid 14, a propellant (liquid phase) 32, a dip tube 33, a pressure vessel 34, an openable atomising head (valve) 35, a button 36, an electromagnetic valve 37, an electromagnetic-valve control circuit 38, a timer circuit 39, and a battery 16 as a power source. The button 36, the electromagnetic valve 37 and the electromagnetic-valve control circuit 38 constitute the control mechanism of open-close of the atomising head 35. The timer circuit 39 serves as a control mechanism of spraying time interval and spraying time. Incidentally, the chemical liquid 14 is dissolved in the propellant 32.

A signal intermittently generated by the timer circuit 39 is applied to the electromagnetic-valve control circuit 38 for controlling an operation of the electromagnetic valve 37. Pressing the button 36 by operating the electromagnetic valve 37 opens the atomising head 35, whereby the chemical liquid 14 together with the propellant 32 flow through the dip tube 33 to be sprayed from an emitting port 41.

### Piezoelectric Method

As other means for carrying out the present invention, there may be used the ultrasonic liquid atomising technique employing the piezoelectric actuator which has been conventionally known to the art. Such a liquid atomising technique is preferred in that the liquid can be atomised without applying heat.

Unlike the aerosol method, this method can atomise the liquid through vibration caused by the electric signal and does not require the pressure vessel nor the propellant contained therein. Accordingly, the method advantageously contributes to a reduced size of the chemical-liquid atomiser apparatus. More advantageously, a further size reduction of the apparatus can be achieved by the piezoelectric atomising head comprising a piezoelectric actuator, a porous diaphragm bonded to the piezoelectric actuator, and a chemical liquid feeding means for supplying a chemical liquid to the diaphragm.

In the piezoelectric method, an output power for atomisation is not particularly limited. The output power is preferably 3 W or less, while a frequency preferably ranges from 20 to 600 kHz, and more preferably from 100 to 600 kHz.

For further reduction of energy required for the atomisation, it is preferred that 1) the diaphragm has an even smaller size and includes pores arranged even more closely, and that 2) the diaphragm is further reduced in thickness, to an extent that a required spraying is ensured.

The concentration of the effective ingredient in the chemical liquid is not particularly limited as long as an effective amount thereof can be emitted in the air. More specifically, the concentration of the effective ingredient in the chemical liquid preferably ranges from 0.02 to 10% (W/V), more preferably from 0.5 to 7.0% (W/V) and particularly preferably from 1.0 to 4.0% (W/V). In a case where a chemical liquid containing an effective ingredient in an extremely low concentration is atomised, it is necessitated that the apparatus is devised for emission of the effective amount of the effective ingredient in the air to increase the size of the diaphragm or to extend the spraying time period, which gives rise to a cause for an increased energy consumption. For the purposes of maintaining the effect of the effective ingredient and saving power, the chemical liquid preferably contains the effective ingredient in a concentration of 0.02% (W/V) or more. On the other hand, many of the effective ingredients themselves exhibit high viscosities at room temperatures. From the viewpoint of suppressing an increase in energy consumption owing to an increase in viscosity of the chemical liquid, the concentration of the effective ingredient in the chemical liquid is preferably 10% (W/V) or less.

Examples of the piezoelectric chemical-liquid atomiser apparatus include the piezoelectric chemical-liquid atomiser apparatus of the present invention, an apparatus disclosed in Japanese Unexamined Patent Publication No. Hei 7-501481 and the like. The atomiser apparatus disclosed in this publication comprises an actuator having an annular disk shape with a hole at a center thereof, and a diaphragm being bonded to the central hole. The publication teaches that the use of a thin electroacoustic actuator favorably contributes to the reduction of costs, power consumption and the size of the overall apparatus.

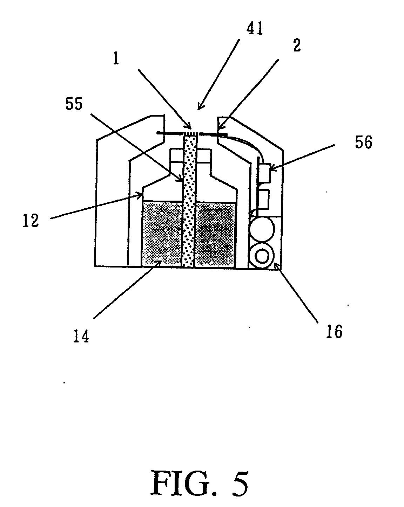

As to the piezoelectric chemical-liquid atomiser apparatus, there may be used an apparatus shown in Figure 5, for example.

Figure 5 is a schematic view illustrating a chemical-liquid atomiser apparatus for the piezoelectric method. In Figure 5, the apparatus comprises a piezoelectric atomising head comprising a piezoelectric actuator 2 and a porous diaphragm 1 indirectly attached to the piezoelectric actuator; a chemical liquid vessel 12 including a wick 55 slightly touching the diaphragm 1; a chemical liquid 14 contained in the chemical liquid vessel 12; an oscillation control circuit 56 connected to the piezoelectric actuator 2; and a battery 16 as a power source. The oscillation control circuit 56 has functions of a piezoelectric oscillation control and a timer control. The chemical liquid 14 in the vessel 12 passes through the wick 55, a chemical-liquid feeding means, to be supplied to the diaphragm 1 slightly touching the wick 55. The diaphragm 1 includes a large number of pores, through which pores the chemical liquid is made into fine particles by vibration of the diaphragm 1, and the resultant fine chemical liquid particles are sprayed from an emitting port 41. The oscillation control circuit 56 controls the spraying time and the spraying time interval to provide the desired intermittent spraying of the liquid. Although not illustrated in Figure 5, the apparatus may have an arrangement to allow a user to arbitrarily set the spraying time and spraying time interval for controlling the amount of sprayed chemical liquid depending upon the desired effect or a space in which the apparatus is used. A method for allowing the user to make arbitrary settings is not particularly limited.

Here, the term "slight touching" between the wick and the diaphragm refers to a state of slight touch in which the wick so softly touches the diaphragm to a degree as not to interfere with the vibration thereof, or to a state where such a fine gap is formed between the wick and the diaphragm that a film of the chemical liquid formed on the top surface of the wick barely contacts the diaphragm.

### EXAMPLES

The present invention will be described in further by means of the following working examples, comparative examples and test examples, without intending to limit the scope or spirit of the present invention thereto.

A piezoelectric chemical-liquid atomiser apparatus similar to that shown in Figure 1 was produced. The chemical-liquid atomiser apparatus was used in Examples 1 to 10 and Comparative Examples 1 to 4.

This apparatus employed a piezoelectric actuator having an annular disk-like shape, and a diaphragm attached to the piezoelectric actuator included a plurality of fine pores in regular arrangement. Here, the pores were constructed such that its diameter was progressively decreased from a chemical-liquid feed surface (back side) toward a chemical-liquid atomising surface (front side).

An AC adapter of DC 3V was used as a power source for driving the piezoelectric actuator. The power was adjusted to a frequency of 113 kHz and a voltage of 43 V in an oscillation control circuit arranged within the apparatus. The piezoelectric actuator was intermittently driven through a timer control comprising an output for 0.5 seconds and a quiescent period of about 29.5 seconds.

### Examples 1 to 10

In the aforesaid chemical-liquid atomiser apparatus, a second chemical-liquid passage portion was fixed in the apparatus body in a manner such that when a chemical liquid vessel was housed in a predetermined position in the atomiser apparatus, one end of a first chemical-liquid passage portion was abutted against one end of the second chemical-liquid passage portion, and the other end of the second chemical-liquid passage portion slight touched a diaphragm. The first chemical-liquid passage portion had a length of 30 mm. The second chemical-liquid passage portion in Example 1 had a length in a horizontal direction of 20 mm, and those of Examples 2 to 10 had a length in a vertical direction of 15 mm. Materials used for the chemical-liquid passage portions, methods for bonding the chemical-liquid passage portions, and methods of fitting the chemical liquid vessel in the atomiser apparatus are shown in Tables 1 and 2. Incidentally, a ceramic, porous material having a relatively low density was used as an inorganic powder sintered body.

In addition, the chemical liquid vessel was provided with a vent having a size of 0.8 mm² at an upper portion of the chemical liquid vessel to make an internal pressure of the vessel constantly equal to an external pressure.

### Comparative Examples 1 to 4

The same chemical-liquid atomiser apparatus and the chemical liquid vessel were used except that in Comparative Example 1 to 4, the wick was not divided, and that the wick was a means for directly supplying the chemical liquid to the diaphragm. The gap between the wick and the diaphragm was so adjusted that the wick slight touched the diaphragm when housing the chemical liquid at a predetermined position in the atomiser apparatus at initial mounting. Materials used for the chemical-liquid passage portions, and methods of fitting the chemical liquid vessel in the atomiser apparatus are shown in Tables 1 and 2. Incidentally, a wick having a high density was used as an inorganic powdery binder material in Comparative Example 4, the wick prepared by binding an inorganic powders, such as clay talc, and diatomaceous earth generally employed in liquid heat transpiration agents with an organic binder.

In addition, the chemical liquid vessel was provided with a vent having a size of 0.8 mm² at an upper portion of the chemical liquid vessel.

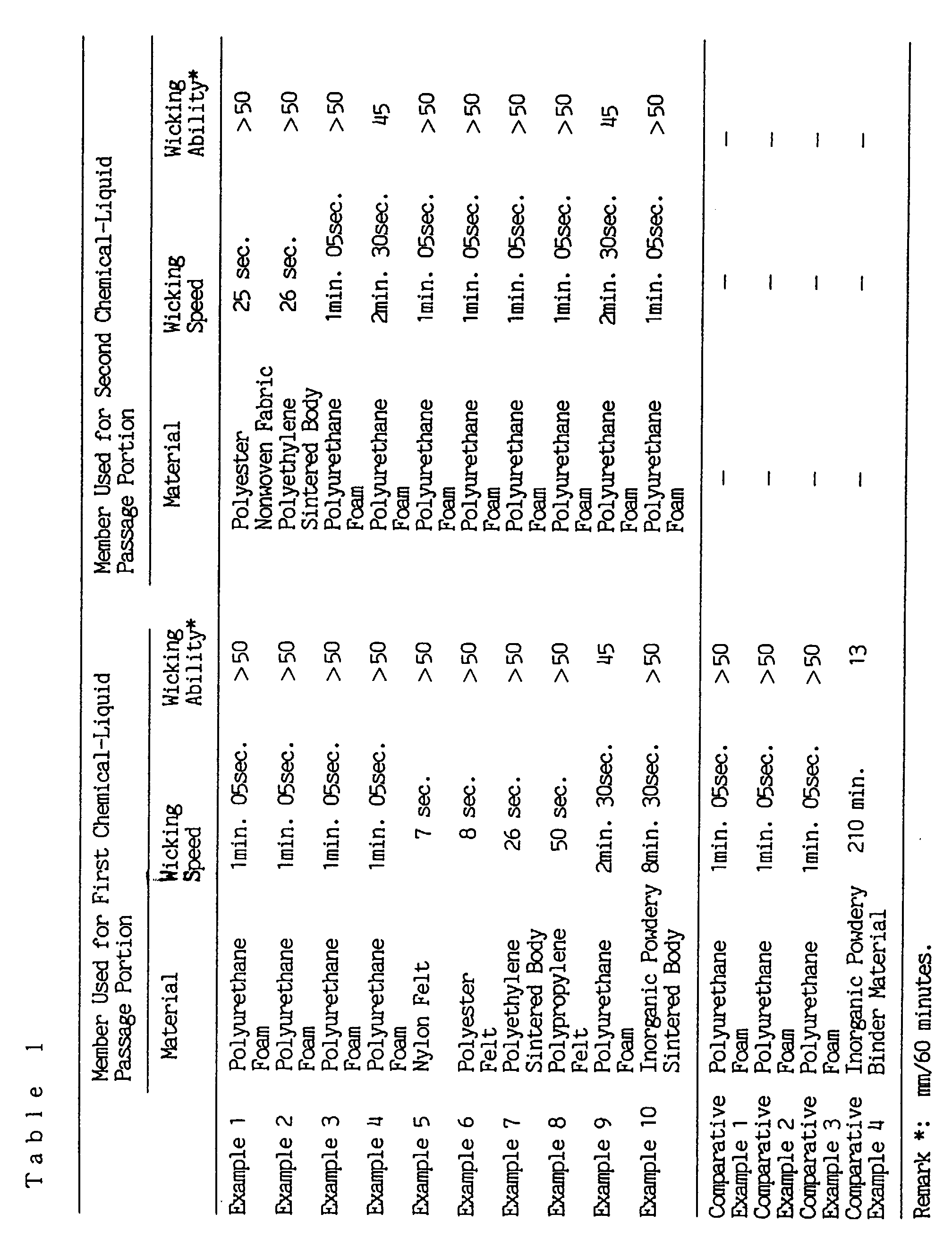

**Table 2**

| | Method of Bonding Chemical-Liquid Passage Portions | Method of Fitting Chemical Liquid Vessels |
|---|---|---|
| Example 1 | Type A | Lateral Snap-Fit |
| Example 2 | Type B | Bottom-Top Housing |
| Example 3 | Type D | Lateral Snap-Fit |
| Example 4 | Type D | Lateral Snap-Fit |
| Example 5 | Type D | Lateral Snap-Fit |
| Example 6 | Type D | Lateral Snap-Fit |
| Example 7 | Type D | Lateral Snap-Fit |
| Example 8 | Type D | Lateral Snap-Fit |
| Example 9 | Type D | Lateral Snap-Fit |
| Example 10 | Type D | Lateral Snap-Fit |
| Comparative Example 1 | - | Lateral Snap-Fit |
| Comparative Example 2 | - | Bottom-Top Housing |
| Comparative Example 3 | - | Bottom Screw Housing |
| Comparative Example 4 | - | Lateral Snap-Fit |

As to the methods of bonding the first and second chemical-liquid passage portions, Type A refers to a method shown in Figure 1, A1 and A2; Type B refers to a method shown in Figure 1, B1 and B2; and Type D refers to a method shown in Figure 2.

### Comparative Example 5

The conventionally used liquid, heat transpiration apparatus was used in Comparative Example 5. The wick was an inorganic powdery binder material. Owing to the shape of the vessel, the wick had a length of 30 mm, and it was so constructed that a polyethylene sintered body was connected to a bottom portion of the wick housed inside the chemical liquid vessel. Also, the liquid, heat transpiration apparatus was constructed that a PTC heating member was heated by applying AC 100 V, and an upper portion of the wick was heated in an indirect manner to carry out transpiration of the chemical liquid.

### Test Example 1

A chemical liquid atomisation test was carried out by using the apparatuses of Examples 1 to 4 and of Comparative Examples 1 to 3.

A chemical liquid vessel was housed in the apparatus body, and the apparatus was operated for 24 hours to determine an initial amount of the atomised chemical liquid. Subsequently, the chemical liquid vessel was removed from the apparatus body and then was housed therein again. This process was repeated. After this process was repeated by a predetermined number of times (10 times, 20 times, 30 times), the apparatus was operated for 24 hours to determine an amount of the atomised chemical liquid. An amount by volume of the atomised chemical liquid per hour was calculated from each weight reduction of the chemical liquid resulting from the operation of the apparatus for 24 hours.

The chemical liquid used in Test Example 1 was an n-paraffin solution containing d•d-T80-prallethrin (Etoc) at a concentration of 2.67% (w/v) as an effective ingredient. The n-paraffin consisted essentially of an aliphatic saturated hydrocarbon having 14 carbon atoms.

In this test, an initial amount of the atomised liquid per hour was set to at about 30 µL.

The results are shown in Table 3.

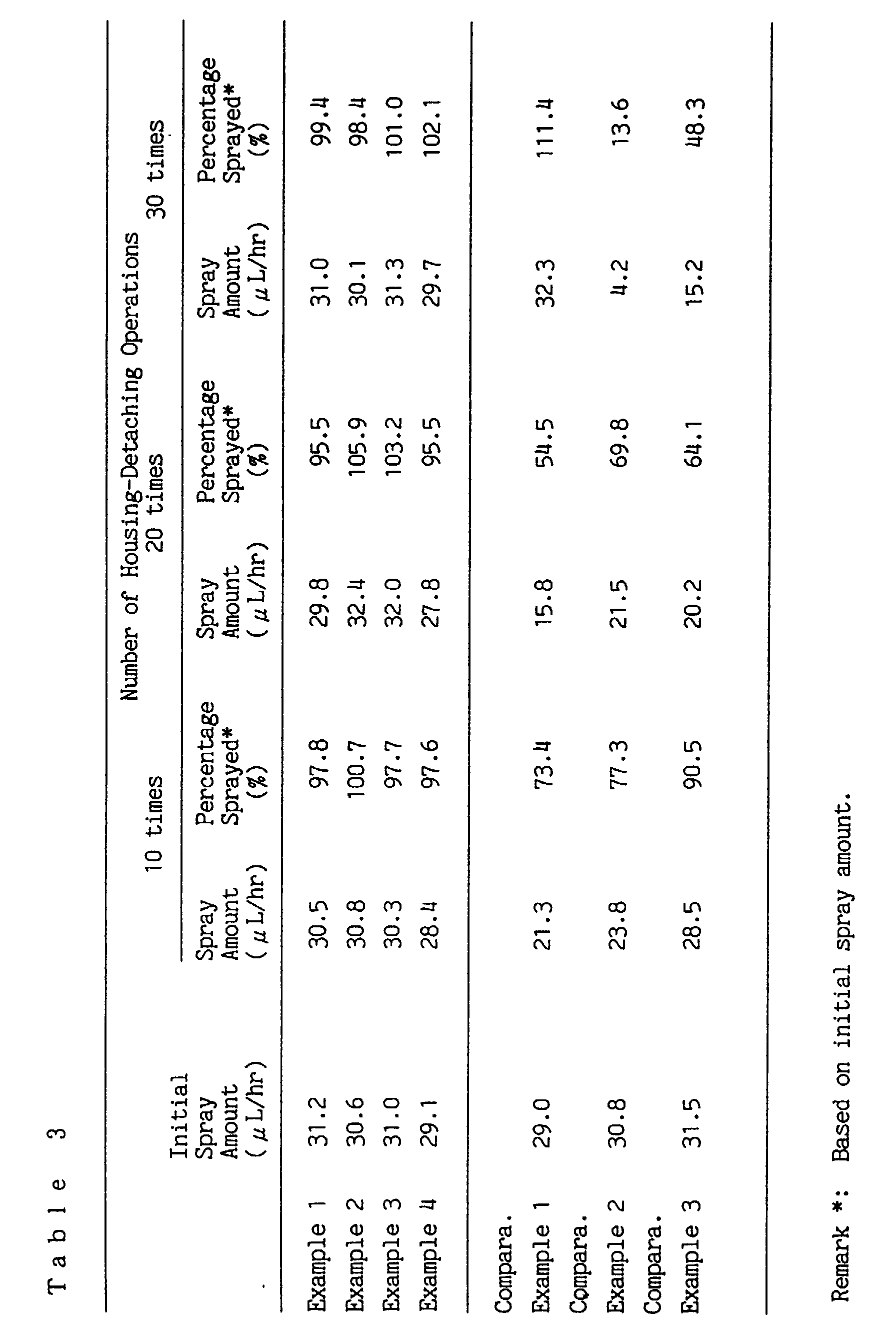

It is clear from Table 3 that the apparatuses of the present invention provide quite stable amounts of liquid atomised regardless of the state of the chemical liquid vessel being mounted and detached. By contrast, there are great variations in the amount of liquid atomised by the apparatuses of Comparative Examples caused by the mounting and detaching of the chemical liquid vessels. This is presumably owing to the fact that the apparatuses of the present invention maintain a constant state of contact between the diaphragm and the wick regardless of the state of the chemical liquid vessel being mounted or detached, whereas the apparatuses of the comparative examples cause changes in the state of contact between the diaphragm and the wick for each mounting and detaching of the chemical liquid vessel.

### Test Example 2

Atomisation or transpiration of a chemical liquid was carried out using the apparatuses of Examples 3 to 10 and of Comparative Examples 4 and 5. There was determined an amount of discharge of an effective ingredient after each lapse of a predetermined time period (10 hours, 300 hours, 600 hours, 900 hours, 1200 hours) from the initiation of atomisation or transpiration. The results are shown in Table 4.

The mode of atomisation was carried out in an intermittent manner, wherein a cycle of spraying for about 0.5 seconds and quieting for about 29.5 seconds was repeatedly carried out. The initial amount of the atomised liquid per hour was set to about 30 µL. Here, in the apparatus and the chemical liquid vessel of Comparative Example 4, the apparatus was operated in the same manner as that of Comparative Example 1 except for that only the material for the first chemical-liquid passage portion was changed.

The amount of discharge of an effective ingredient was determined as follows. Specifically, sprayed or transpired chemical liquid including the effective ingredient was collected under suction with a silica gel, and the collected components were subjected to acetone extraction. The resulting extract was subject to quantitative analysis by the gas chromatography thereby to determine an amount of discharge of the effective ingredient.

The chemical liquid used in Test Example 2 was an n-paraffin solution containing d•d-T80-prallethrin (Etoc) at a concentration of 2.67% (w/v). The n-paraffin consisted essentially of an aliphatic saturated hydrocarbon having 14 carbon atoms.

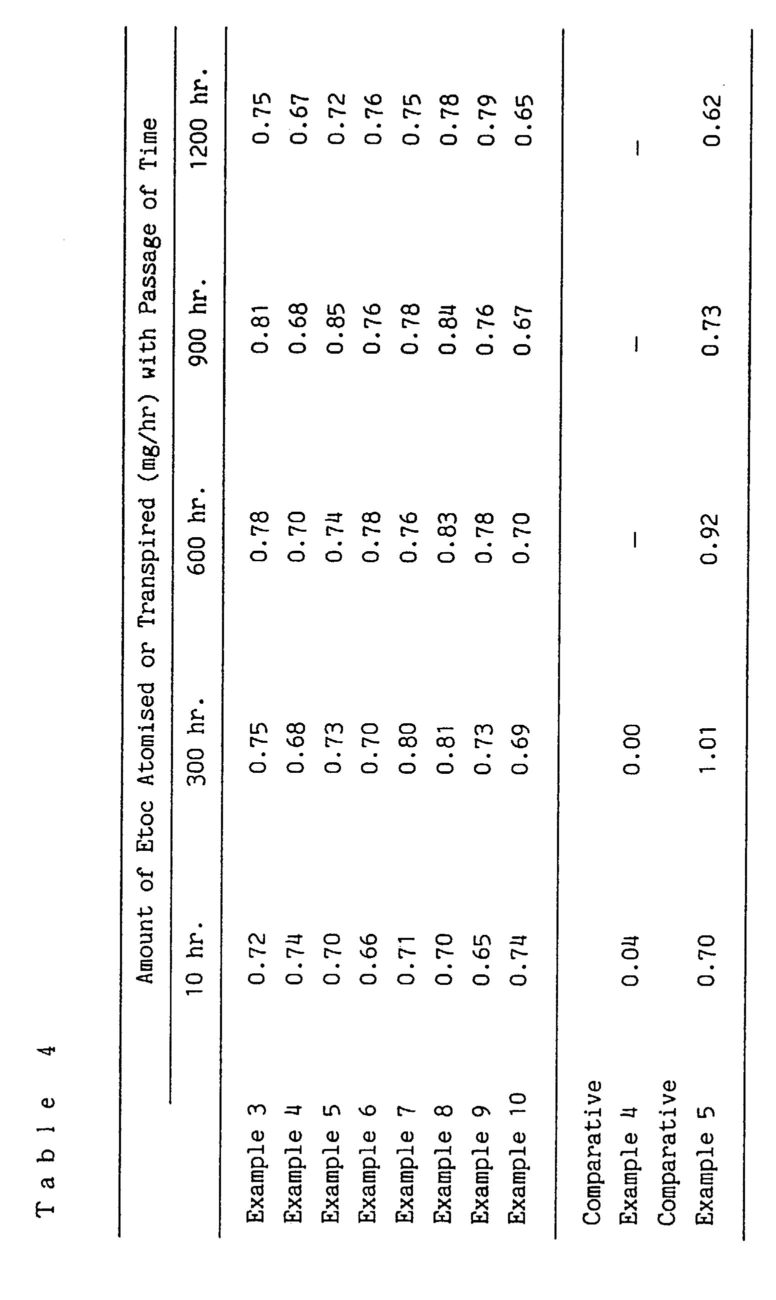

It is clear from Table 4 that the amount of discharge of the effective ingredient in Example 3 to 10 is stable for a long period of time from the initiation of the operation of the apparatus, and even stabler in the discharge of the effective ingredient than the conventional liquid, heat transpiration method in Comparative Example 5. In Comparative Example 4, there arises a problem in spraying even from the initial stage of spraying, so that no effective ingredients are discharged with the passage of time.

### Examples 11 to 44 and Comparative Examples 6 to 11

As the effective ingredients, there were used d•d-T80-prallethrin (Etoc), d1•d-T80-allethrin (Pynamin Forte, simply referred hereinafter as "Pynamin f"), terallethrin (Knockthrin), d-T80-phthalthrin (Neo-Pynamin Forte, simply referred hereinafter as "Neo-Pynamin f"), and d-T80-cyphenothrin (Gokilaht). For each of effective ingredients, the size of fine chemical liquid particles, the spraying time interval, the amount of each effective ingredient atomised per hour were adjusted as shown in Tables 5 and 6 to carried out the test. Incidentally, the tables indicate the particle sizes in a simplified manner as described below. More specifically, 90% of particles (X-90) based on the volume cumulative distribution (volume cumulative percent) had a size of N µm, sizes of the particles were simply referred to "N µm." The spraying time interval was defined as a period between the start of a first atomisation and the start of the subsequent atomisation. The spraying time was set to be shorter than the spraying time interval.

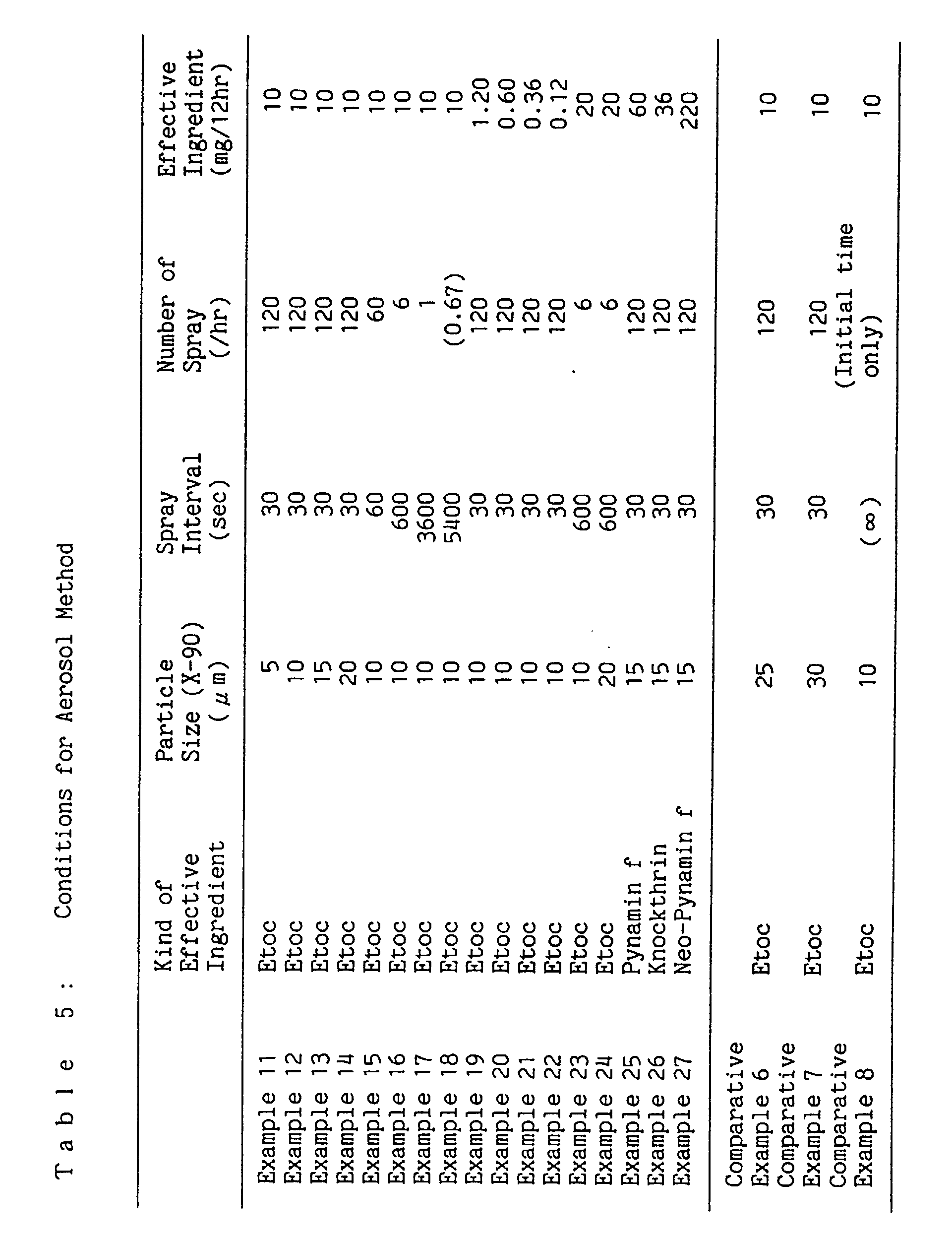

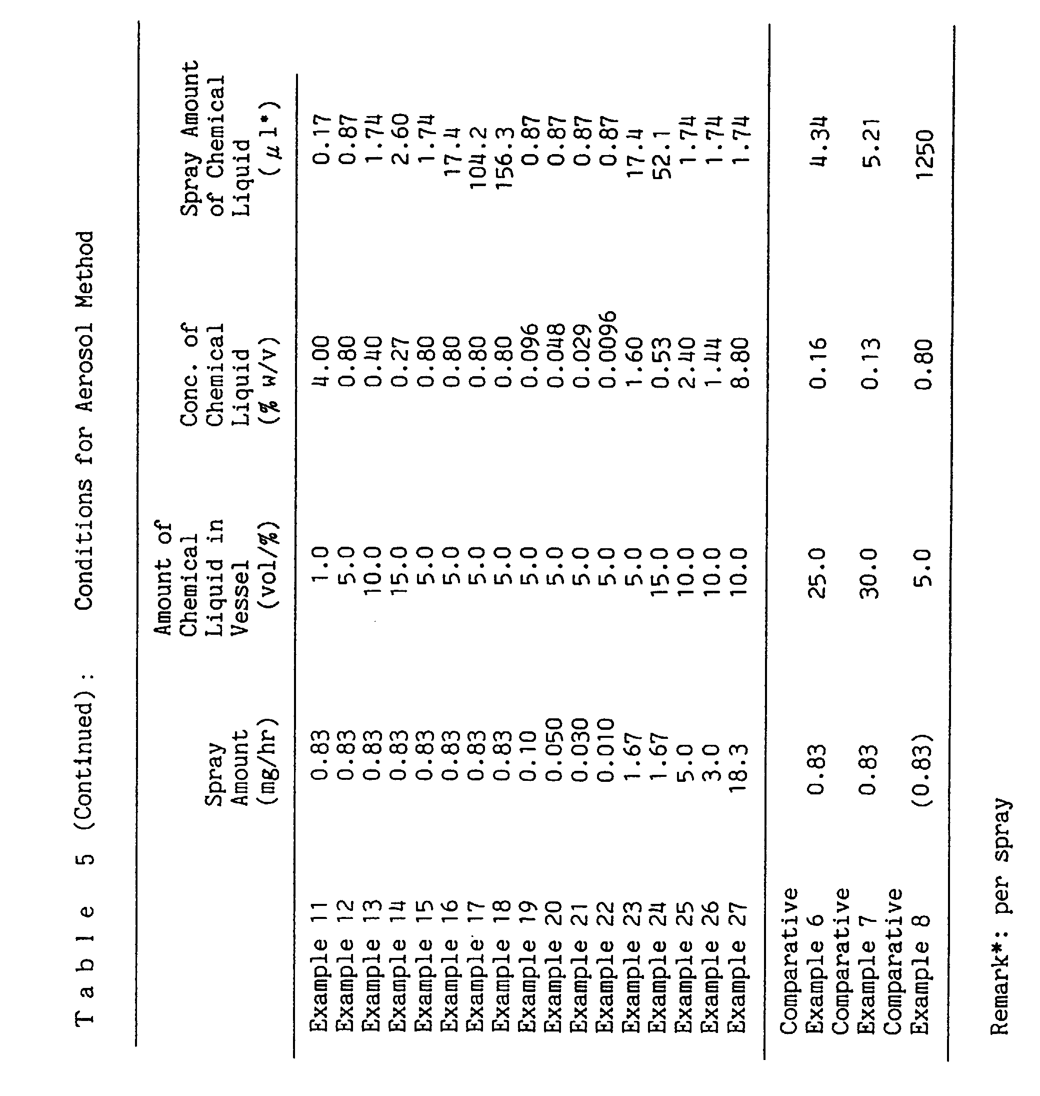

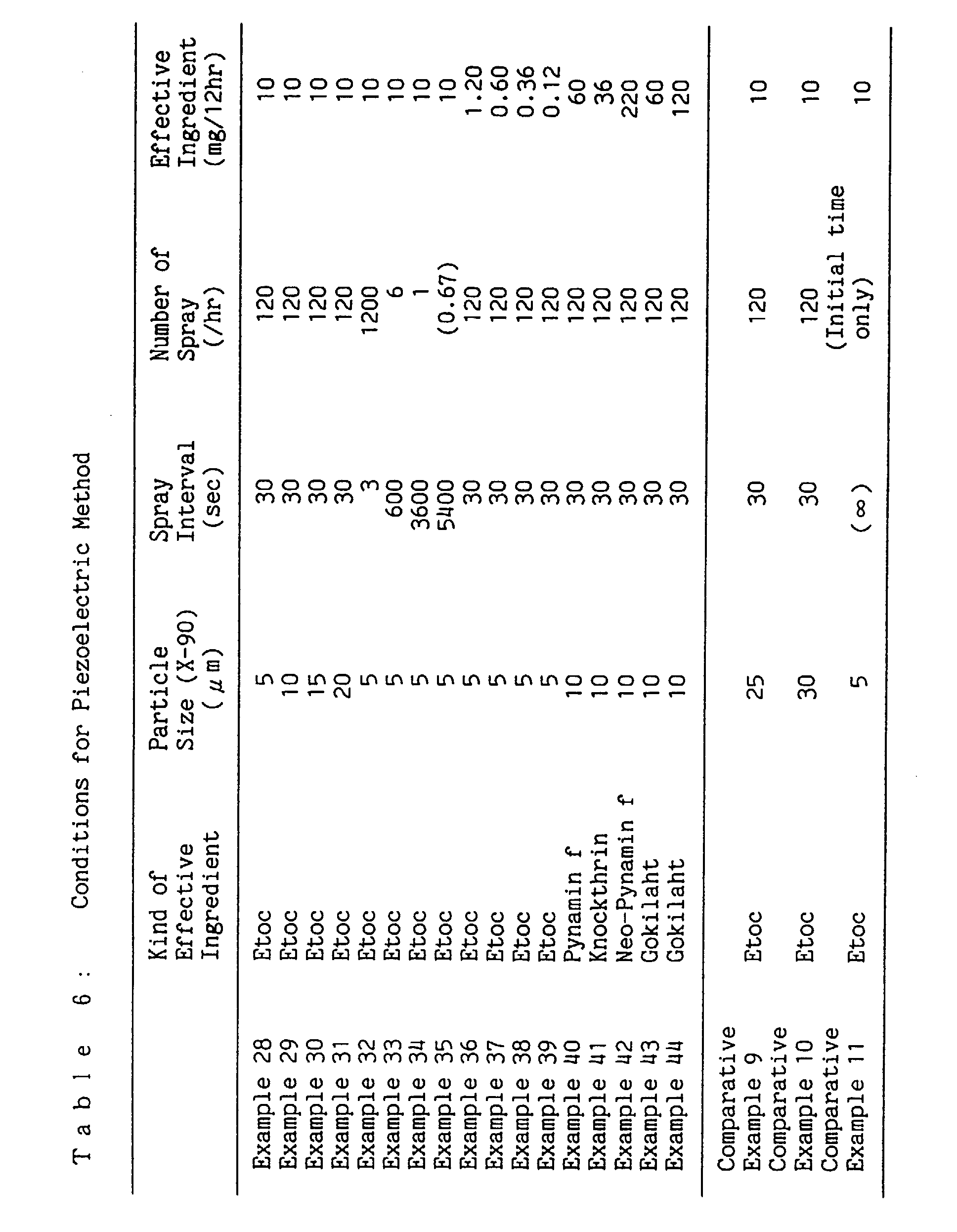

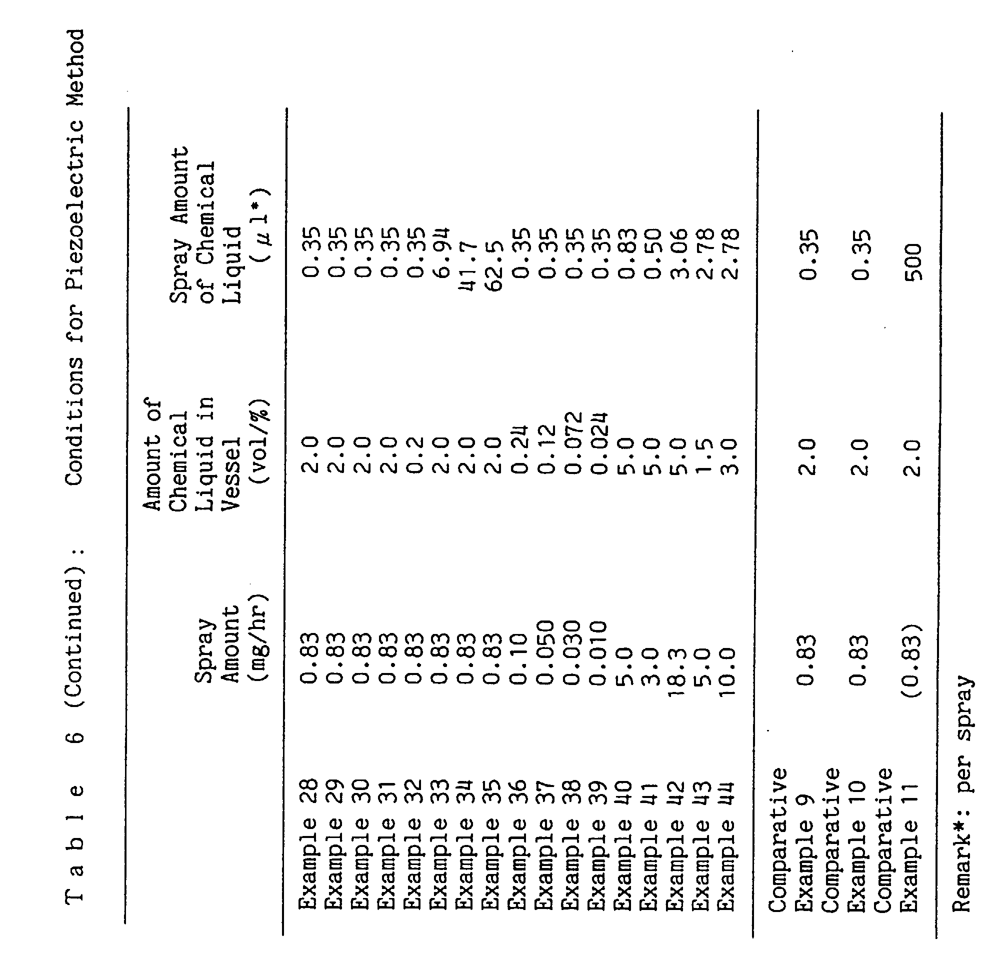

### Aerosol Method

An initial volume ratio of a chemical liquid was set to be within a range of 1.0 to 30% to a pressure vessel of 300 mL under the conditions given at Table 5. The chemical liquid was prepared by using ethanol as a solvent. In addition, DME was used as a propellant, and an internal pressure of the pressure vessel was adjusted to be from about 4 to about 5 kg/cm².

The diameter of the fine chemical liquid particles could be adjusted by a suitable type of valves and spray buttons to be used. In the present invention, it was adjusted by varying the volume ratio of the chemical liquid to the volume of the pressure vessel, the chemical liquid comprising the effective ingredient and a solvent. For example, when 90% of discharged chemical liquid particles based on the volume cumulative distribution had a size of about 5 µm, an initial volume ratio of the chemical liquid to the pressure vessel was set to be 1%. Similarly, when 90% of particles had a size of about 10 µm, an initial volume ratio of the chemical liquid was set to be 5%; when 90% of particles had a size of about 15 µm, an initial volume ratio of the chemical liquid was set to be 10%; when 90% of particles had a size of about 20 µm, an initial volume ratio of the chemical liquid was set to be 15%; when 90% of particles had a size of about 25 µm, an initial volume ratio of the chemical liquid was set to be 25%; and when 90% of particles had a size of about 30 µm, an initial volume ratio of the chemical liquid was set to be 30%.

As to the amount of discharge of the chemical liquid per one spray, the concentration of each of the effective ingredient in the chemical liquid was adjusted as follows. For example,

| | |
|---|---|
| d•d-T80-prallethrin (Etoc) | 0.0096 to 4.0% (w/v) |
| d1•d-T80-allethrin (Pynamin f) | 2.4% (w/v) |
| terallethrin (Knockthrin) | 1.44% (w/v) |
| d-T80-phthalthrin (Neo-Pynamin f) | 8.8% (w/v) |

At the same time, a valve-opening time was controlled.

Incidentally, the apparatus shown in Figure 4 was used as an atomiser apparatus in this test.

### Piezoelectric Method

An atomiser chemical liquid was prepared by dissolving an effective ingredient in a solvent. Used as a solvent was n-paraffin essentially consisting of aliphatic saturated hydrocarbon having 12 carbon atoms. Each chemical liquid was prepared so that the effective ingredient was contained in a given concentration. For example, a concentration of d•d-T80-prallethrin (Etoc) was in a range from 0.024 to 2.0% (w/v); each of concentrations of d1•d-T80-allethrin (Pynamin f), terallethrin (Knockthrin) and d-T80-phthalthrin (Neo-Pynamin f) was 5% (w/v); and a concentration of d-T-80-cyphenothrin (Gokilaht) was 1.5% or 3% (w/v).

The diameter of the chemical liquid particles was adjusted by changing a size of the pores provided on the diaphragm. Specifically, the desired particle size was obtained by adjusting the pore diameter to be from about 1 to 13 µm regularly arranged on the diaphragm, the diaphragm being bonded to the piezoelectric actuator. For instance, when 90% of discharged chemical liquid particles based on the volume cumulative distribution had a size of about 5 µm, the size of pores provided on the diaphragm was set to be about 1 to about 3 µm. Similarly, when 90% of particles had a size of about 10 µm, the size of pores was set to be about 3 to about 5 µm; when 90% of particles had a size of about 15 µm, the size of pores was set to be about 4 to about 7 µm; when 90% of particles had a size of about 20 µm, the size of pores was set to be about 6 to about 9 µm; when 90% of particles had a size of about 25 µm, the size of pores was set to be about 8 to about 11 µm; and when 90% of particles had a size of about 30 µm, the size of pores was set to be about 9 to about 13 µm.

As to the amount of discharge of the chemical liquid per one spray, the amount was adjusted as follows by controlling the vibration period of the piezoelectric actuator. For example,

| | |
|---|---|
| Etoc | 0.35 to 41.7 µL |
| Pynamin f | 0.83 µL |
| Knockthrin | 0.5 µL |
| Neo-Pynamin f | 3.06 µL |
| Gokilaht | 2.78 µL |

Incidentally, the apparatus shown in Figure 5 was used as an atomiser apparatus in this test. In the oscillation control circuit, DC 3V was used as a power source to increase the voltage to 43 V, and the piezoelectric actuator was driven while controlling the frequency to 113 kHz.

### Test Example 3

This test was conducted under the setting conditions respectively specified for Examples 11 to 14 and 28 to 31 and Comparative Examples 6, 7, 9 and 10 as shown in Tables 5 and 6. The size of 90% of particles contained in the discharged chemical liquid particles based on the volume cumulative distribution was varied in a range from about 5 µm to about 30 µm to evaluate a knockdown effect for particles of respective sizes. As a sample insect, 100 female imagines of *Culex pipiens pallens* were released in a test room of about 8-tatami, i.e. about 30 m³, and the number knocked down insects was counted with the passage of time from release. From the results obtained, KT₅₀ values calculated according to Bliss' Probit Method were compared. The KT₅₀ value represented a time period required for knocking down 50% of the sample insects. Hence, the smaller the KT₅₀, the greater the knockdown effect, i.e. the effect for repelling or eliminating a target harmful organism.

Measurement points to count the knocked down insects were set to times after 30 minutes, one hour, 3.5 hours, 7 hours, and 12 hours from the start of the test. In the piezoelectric method, an additional measurement point was set to a time after 300 hours from the start of the test. The evaluation of each case was made in the manner aforementioned.

A degree of pollution of the room floor with the chemical liquid was evaluated through a visual inspection after the operation under each condition. The inspection was performed after a lapse of 120 hours from the start of the test with respect to the aerosol method and after a lapse of 360 hours with respect to the piezoelectric method. The following evaluations were made for each rank:
○: Pollution of a degree that the floor was not changed in color or slightly changed;
Δ: Pollution of a degree that the floor was somewhat changed in color or became somewhat greasy; and
x: Pollution of a degree that the floor became greasy. The results are shown in Tables 7 and 8.

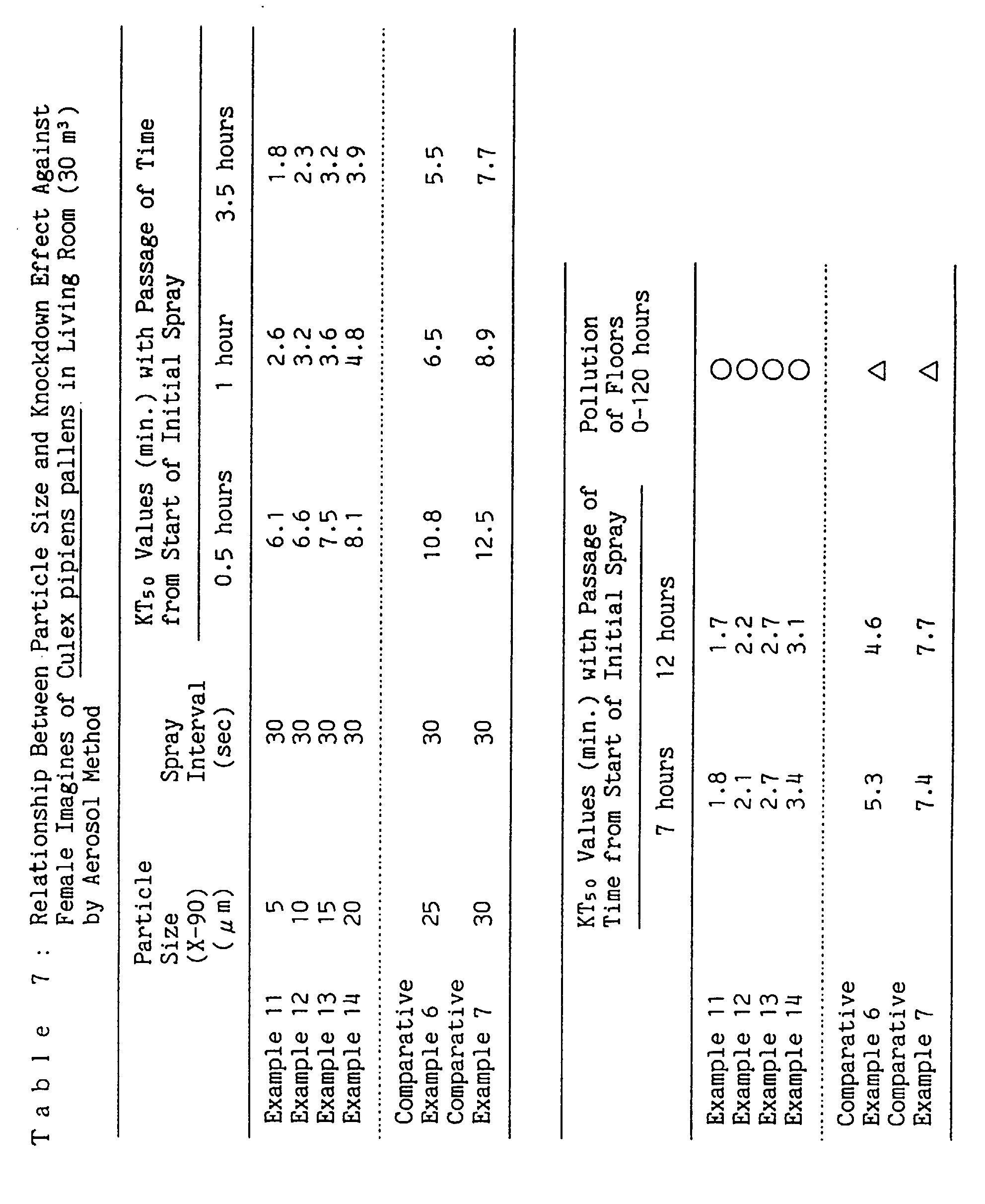

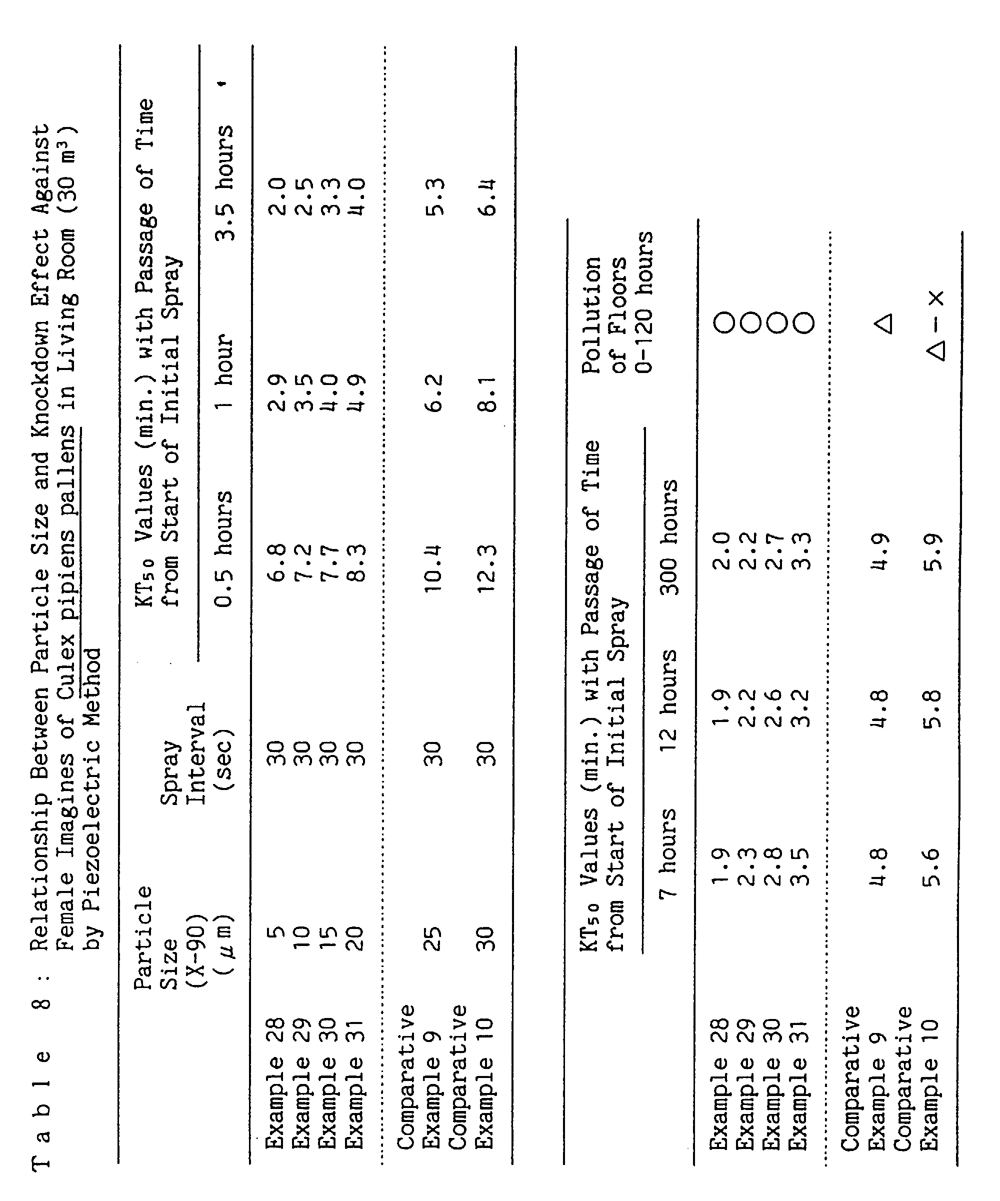

It is clear from the results that smaller the particle size, larger the knockdown effect and earlier the time in which the effect from the start of spraying was exhibited. Particularly when 90% of discharged chemical liquid particles based on the volume cumulative distribution have a size of about 20 µm or less, the most effective knockdown effect can be confirmed practically.

### Test Example 4

This test was conducted under the setting conditions respectively specified for Examples 15 to 18 and 32 to 35 and Comparative Examples 8 and 11 as shown in Tables 5 and 6. The size of 90% of discharged chemical liquid particles based on the volume cumulative distribution was about 10 µm for the aerosol method, and about 5 µm for the piezoelectric method to evaluate a knockdown effect for each spraying interval under conditions that spraying intervals was set between 3 and 5400 sec.

The method for evaluating the knockdown effect and the degree of pollution on the floor were carried out by method described in Test Example 3.

The results are shown in Tables 9 and 10.

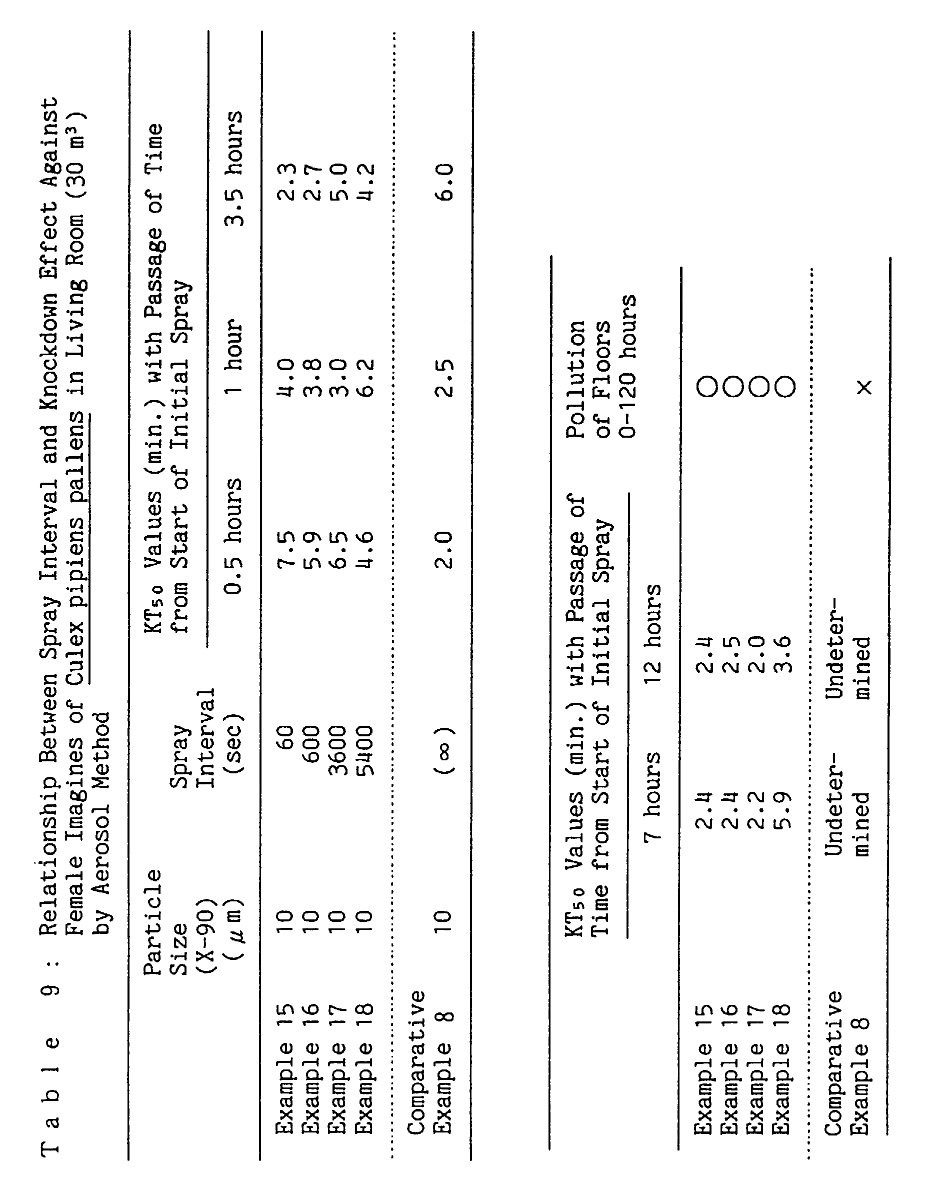

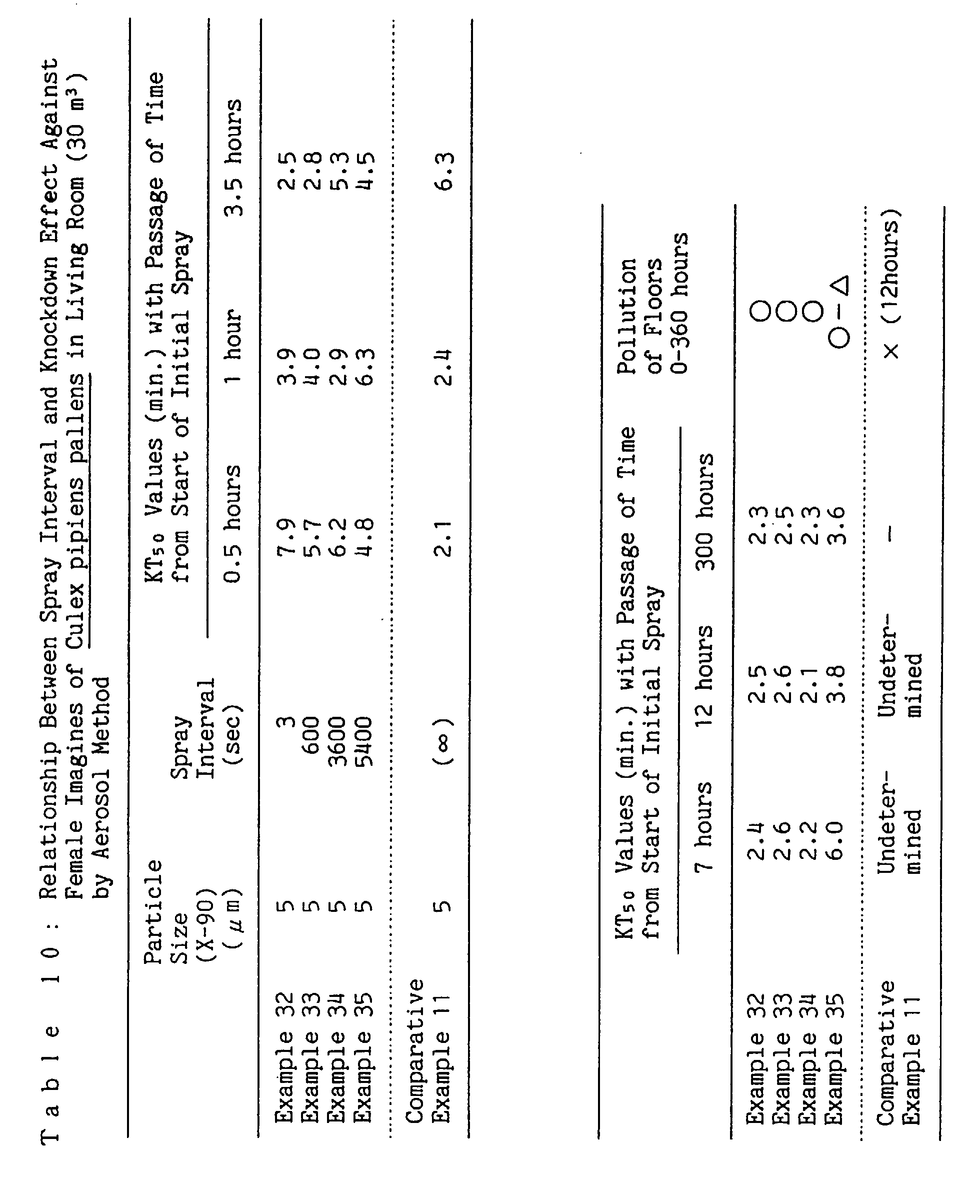

It is clear from the results that although a knockdown effect can be obtained when the spraying interval of the chemical liquid was set at 3600 seconds, namely 60 minutes, depending upon the duration of the passage of time, there are observed deviations among the knockdown effect in immediately after spraying and in subsequent spraying. For instance, when spraying interval is 5400 sec., deviations tend to become large, so that the spraying interval is preferably within 3600 sec.

In addition, it is clear from Comparative Examples 8 and 11 that although the total amount of the effective ingredient until spraying for 12 hours is at the same level as Examples, the effect cannot be sustained for a long period of time, thereby showing usefulness in an intermittent spraying in an appropriate interval.

### Test Example 5

This test was conducted under the setting conditions respectively specified for Examples 13, 25 to 27, 29, and 40 to 42 as shown in Tables 5 and 6 to evaluate a knockdown effect for different effective ingredients included in the chemical liquid particles released. The method for evaluating the knockdown effect was carried out by method described in Test Example 3.

The results are shown in Table 11.

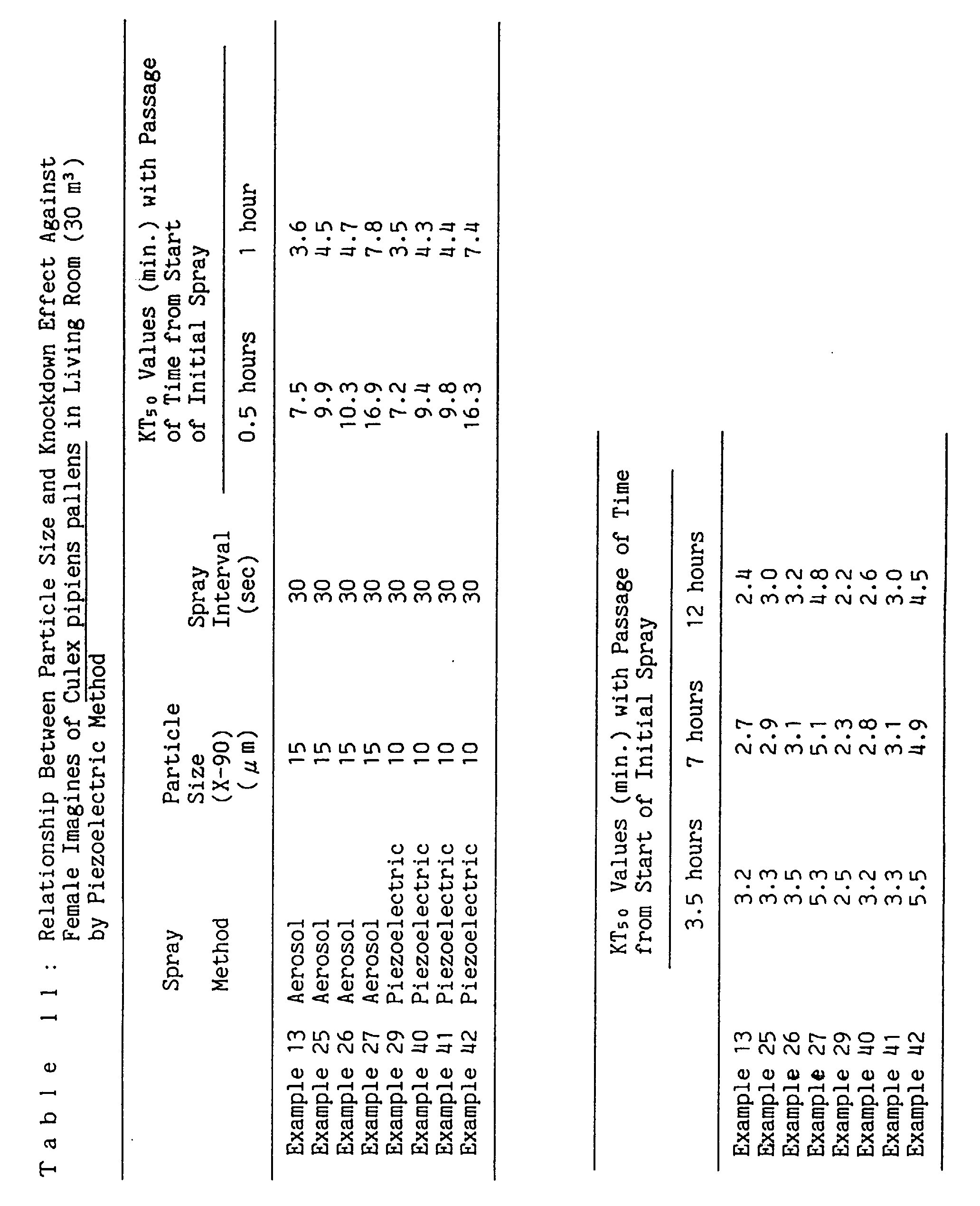

It is clear from the results that even if the effective ingredients were different, the effect activity owned by the effective ingredient itself can be exhibited, suggesting that an appropriate effect can be obtained by adjusting the amount of the effective ingredient sprayed depending upon the effect activity.

### Test Example 6

This test was conducted under the setting conditions respectively specified for Examples 23 and 24 as shown in Table 5 to evaluate a knockdown effect to female imagines of *Culex pipiens pallens* in a 16-tatami mat room (about 60 m³) when a spraying amount of the chemical liquid per spray was set for a size of 60 m³. The method for evaluating the knockdown effect was carried out by method described in Test Example 3.

The results are shown in Table 12.

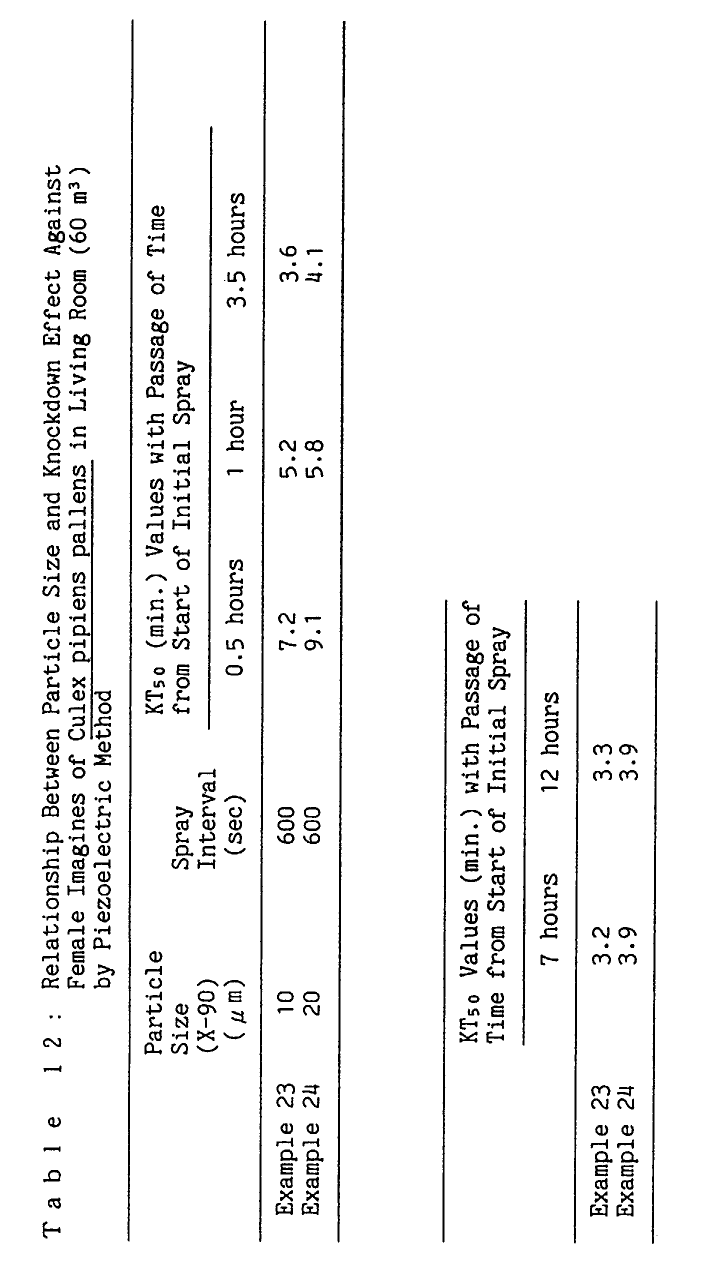

It is clear from the results that by doubling the spray amount of the chemical liquid of that used for 30 m³ room, desired effects can be obtained when doubling its volume.

### Test Example 7

This test was conducted under the setting conditions respectively specified for Examples 19 to 22 and 36 to 39 as shown in Table 5, and studying on the relationship between the released amount of the effective ingredient per unit time and unit space and effect of inhibiting blood sucking of female imagines of *Aedes albopictus* after two hours from the start of test. The effect of inhibiting blood sucking was evaluated as follows. A living room-testing room was made ready by placing a rat fixed to a wire net as a source of blood sucking in the middle of a living area of 8-tatami mat room (about 30 m³), and the chemical liquid was intermittently sprayed over a period of 2 hours. Next, 100 individuals of female imagines of *Aedes albopictus* were released, and the total number of blood sucking after two hours from release was counted. In addition, as a control, a room without spraying the chemical liquid was prepared, and the number of blood sucking was counted.

The results are shown in Table 13.

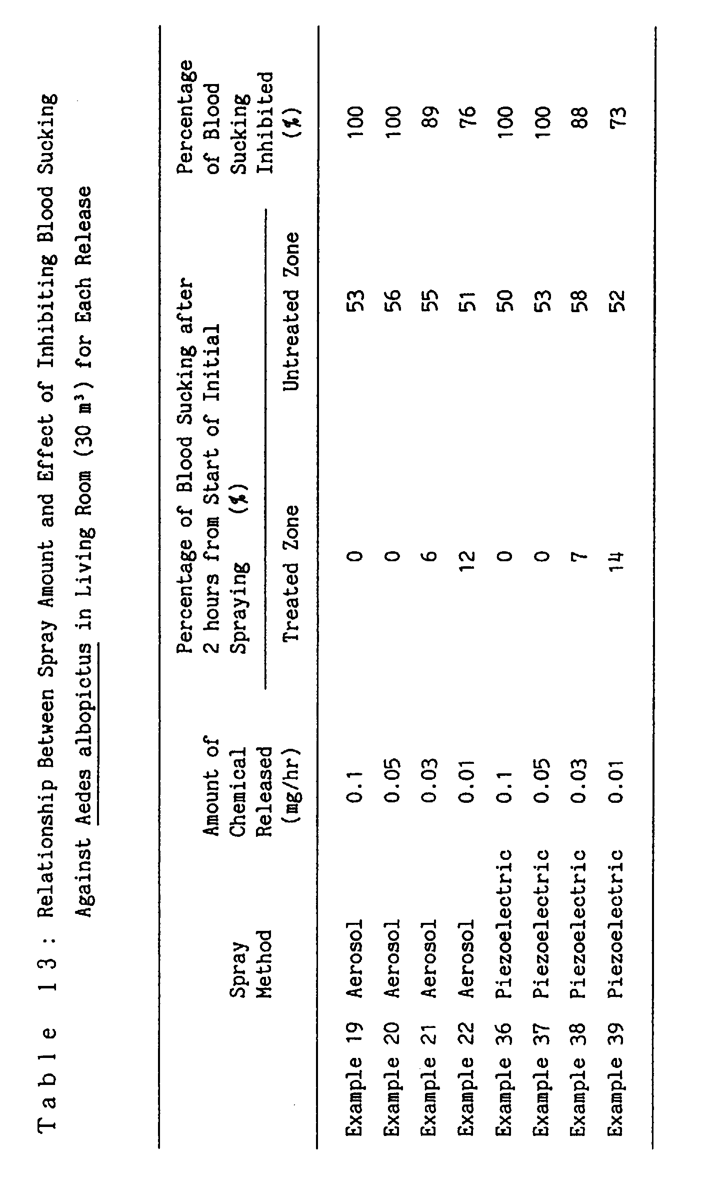

It is clear from the above test that in order to obtain a minimal level of effect of inhibiting blood sucking for preventing the harm of blood sucking by mosquitos, the release amount of the effective ingredient per one hour per 30 m³ is desirably to be set at 0.01 mg or more.

### Test Example 8

This test was conducted under the setting conditions respectively specified for Examples 43 and 44 as shown in Table 5, in which the subject to be tested as harmful organisms was cockroaches. Incidentally, the test was carried out against *Periplaneta fuliginosa* and *Blattella germanica* in a 8-tatami mat room (equivalent to a space of about 30 m³). At four corners of the room were placed polyethylene cups containing subject insects to which vaseline was applied in the inner surface of the cup to avoid escape, and a box-shaped cover having dimensions of 30 cm x 30 cm x 48 cm with a hole of 6 cm x 6 cm on its side was placed thereon. Measurement points to count the knocked down insects were set to times after 3 hours, 6 hours, and 12 hours from the start of the test.

The results are shown in Table 14.

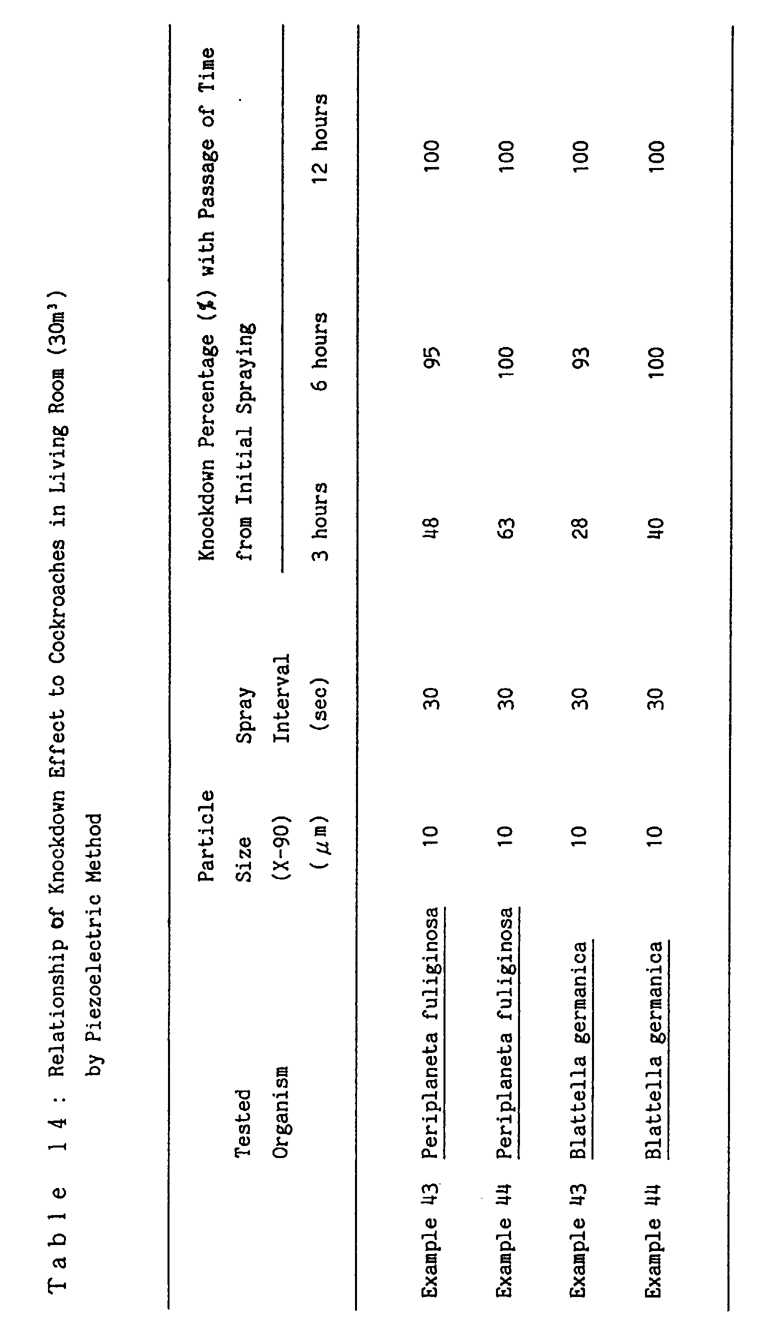

It is clear from the above test that its effect is also confirmed against cockroaches, showing that similar usefulness to mosquitoes can be found for harmful organisms other than mosquitoes.

Since the piezoelectric chemical-liquid spraying apparatus of the present invention has easy exchange operation of the chemical liquid, having substantially no liquid leakage upon chemical liquid exchange, and having excellent spraying stability. In addition, according to the present invention, since the chemical liquid can be intermittently sprayed, the remarkable reduction in energy consumption and in the amount of the chemical liquid used can be achieved. Moreover, since batteries, etc. can be used, the method of the present invention has a wide range of applicability.

## Claims

1. A piezoelectric chemical-liquid atomiser apparatus comprising:
a chemical liquid vessel detachably housed in the atomiser apparatus, and
a wick for supplying a chemical liquid therethrough to a piezoelectric atomising head arranged in the atomiser apparatus,
characterized in that:
the wick is divided into a first chemical-liquid passage portion and a second chemical-liquid passage portion, wherein
(A) the first chemical-liquid passage portion is arranged within the chemical liquid vessel, one end thereof contacting the chemical liquid and the other end thereof being abutted against one end of the second chemical-liquid passage portion; and
(B) the second chemical-liquid passage portion is arranged at a position where the other end thereof slightly touches the piezoelectric atomising head or a position where the other end thereof contacts the piezoelectric atomising head, thereby allowing the chemical liquid to be supplied up to the piezoelectric atomising head through the first chemical-liquid passage portion and the second chemical-liquid passage portion.

2. The piezoelectric chemical-liquid atomiser apparatus according to claim 1, wherein a member in the piezoelectric atomising head which slightly touches the other end of said second chemical-liquid passage portion or contacts the other end of said second chemical-liquid passage portion is any one of a diaphragm, a thin sheet, and a piezoelectric actuator.

3. The piezoelectric chemical-liquid atomiser apparatus according to claim 1 or 2, wherein said first chemical-liquid passage portion and/or said second chemical-liquid passage portion are made of porous materials having continuously permeable pores, resinous materials having continuous foams, and aggregates of resinous fibers.

4. The piezoelectric chemical-liquid atomiser apparatus according to any one of claims 1 to 3, wherein the member usable for the first chemical-liquid passage portion has a wicking speed of the chemical liquid of within 10 minutes.

5. The piezoelectric chemical-liquid atomiser apparatus according to any one of claims 1 to 4, wherein at least one member of the first chemical-liquid passage portion and the second chemical-liquid passage portion is compressed by bringing said first chemical-liquid passage portion into abutment against said second chemical-liquid passage portion.

6. The piezoelectric chemical-liquid atomiser apparatus according to any one of claims 1 to 5, wherein the chemical liquid vessel has a vent with an area of aperture of 1 mm² or less.

7. The piezoelectric chemical-liquid atomiser apparatus according to any one of claims 1 to 6, wherein a packing member is provided at a peripheral portion in which the first chemical-liquid passage portion is brought into abutment against the second chemical-liquid passage portion.

8. The piezoelectric chemical-liquid atomiser apparatus according to any one of claims 1 to 7, wherein the chemical liquid vessel is housed by a lateral slide-fit method, a lateral snap-fit method, and top-down housing method.

9. A method for repelling and eliminating a harmful organism comprising emitting in the air a chemical liquid containing an effective ingredient as atomised chemical liquid fine particles, characterized in that the atomised chemical liquid is sprayed intermittently, and to have a particle size distribution of the resulting atomized chemical liquid fine particles in which 90% by cumulative volume of the chemical liquid fine particles based on volume cumulative distribution has a particle size of 20 µm or less.

10. The method according to claim 9, wherein a spraying time interval is from 3 seconds to 60 minutes.

11. The method according to claim 9 or 10, wherein an amount of sprayed liquid of an effective ingredient in the chemical liquid per hour is from 0.01 to 20 mg per a space of 30 m³.

12. The method according to any one of claims 9 to 11, wherein the effective ingredient is one or more compounds selected from the group consisting of pyrethroid insecticides, pyrethroid-like insecticides, organic phosphorus insecticides, carbamate insecticides, chloronicotine insecticides, insect growth retardants, and microbicides.

13. The method according to any one of claims 9 to 12, wherein the chemical liquid is sprayed in the air by using an aerosol atomiser apparatus comprising a pressure vessel having an openable spray opening, and including a chemical liquid being sealed therein together with a propellant.

14. The method according to claim 13, wherein the atomiser apparatus further comprises a power source, an open/close control mechanism for the spray opening and a control mechanism of spraying time interval and amount of sprayed liquid.

15. The method according to any one of claims 9 to 12, spraying the chemical liquid in the air using a piezoelectric chemical liquid atomiser apparatus comprising a piezoelectric actuator, a porous diaphragm attached to the piezoelectric actuator, a chemical-liquid feeding means for supplying the chemical liquid to the diaphragm, a piezoelectric oscillation circuit connected to the piezoelectric actuator, a control circuit capable of controlling vibration of the diaphragm for intermittently atomising the chemical liquid, a power source, and the chemical liquid.
